(19) Europäisches Patentamt
European Patent Office
Office européen des brevets

(11) Numéro de publication : **0 466 548 A1**

# (12) DEMANDE DE BREVET EUROPEEN

(21) Numéro de dépôt : **91401740.5**

(22) Date de dépôt : **27.06.91**

(51) Int. Cl.[5] : **C07D 471/04,** C07D 487/04, C07D 471/20, A61K 31/435, A61K 31/55, // (C07D471/04, 221:00, 209:00), (C07D487/04, 223:00, 209:00), (C07D471/20, 221:00, 221:00, 209:00)

(30) Priorité : **27.06.90 FR 9008094**

(43) Date de publication de la demande : **15.01.92 Bulletin 92/03**

(84) Etats contractants désignés : **AT BE CH DE DK ES FR GB GR IT LI LU NL SE**

(71) Demandeur : **ADIR ET COMPAGNIE**
**1 rue Carle Hébert**
**F-92415 Courbevoie Cédex (FR)**

(72) Inventeur : **Levy, Jean**
**18 ter rue Houzeau-Muiron**
**F-51100 Reims (FR)**
Inventeur : **Laronze, Jean Yves**
**276 A boulevard Pommery**
**F-51100 Reims (FR)**
Inventeur : **Devissaguet, Michelle**
**14 boulevard d'Inkermann**
**F-92200 Neuilly Sur Seine (FR)**

(54) **Nouveaux 1,2,3,4,5,6-hexahydroazépino [4,5-b]indoles et 1,2,3,4-tétrahydro Beta carbolines, leurs procédés de préparation et les compositions pharmaceutiques qui les contiennent.**

(57) L'invention concerne les dérivés de formule générale **(I)** :

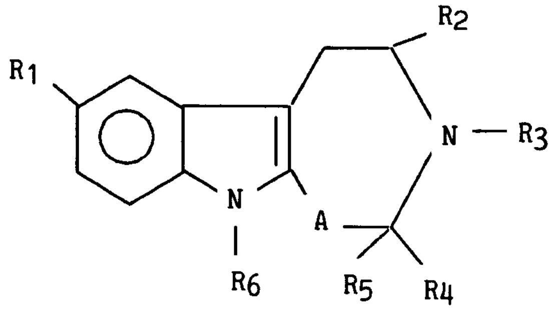

dans laquelle A, $R_1$, $R_2$, $R_3$, $R_4$, $R_5$ et $R_6$ sont tels que définis dans la description.
— leurs isomères, diastéroisomères, énantiomères ainsi que leurs sels d'addition à un acide ou à une base minérale ou organique pharmaceutiquement acceptables
— Médicaments

EP 0 466 548 A1

La présente invention concerne de nouveaux 1,2,3,4,5,6-hexahydroazépino [4,5-b] indoles et 1,2,3,4-tétrahydro β carbolines, leurs procédés de préparation et les compositions pharmaceutiques qui les contiennent.

Les 1,2,3,4,5,6-hexahydroazépino [4,5-b] indoles mentionnés dans l'art antérieur sont tous différents de ceux revendiqués par la demanderesse aucun d'entre eux ne possèdant simultanément un groupement hydroxy, alcoxy ou acétoxy en position 5 avec un ou deux substituants en position 6.

Quelques 5-hydroxy 1,2,3,4,5,6-hexahydroazépino [4,5-b] indoles sont décrits dans la littérature (FR 1524830) comme possédant des propriétés antitussives.

Plus généralement, les 1,2,3,4,5,6-hexahydroazépino [4,5-b] indoles sont le plus souvent décrits comme étant des neuroleptiques, antidépresseurs, sédatifs, tranquillisants et comme moyen de traitement des désordres vasculaires cérébraux (Brevets EP 203902, EP 28381, EP 64317, FR 1524495).

Concernant les 1,2,3,4-tétrahydro β carbolines, si un grand nombre de composés ont été préparés seuls deux produits comportant une fonction carboxylique en 3 et un système spirannique en 1 sont mentionnés dans la littérature sans qu'aucune propriété pharmacologique ne soit revendiquée. Monatsch. Chem. (1985) 116 pp 851-5.

Outre leurs bonnes propriétés sur le système nerveux central, plus particulièrement en tant qu' anxiolytiques, antidépresseurs, antipsychotiques et pour certains d'entre eux leur bonne activité dans le traitement des désordres vasculaires cérébraux et des troubles de la mémoire, certains des composés de l'invention possèdent d'intéressantes propriétés analgésiques, antiinflammatoires, anticonvulsivantes et myorelaxantes que ne possèdent pas les composés de l'art antérieur structuralement les plus proches.

Plus spécifiquement l'invention concerne les indoles substitués de formule générale (I) :

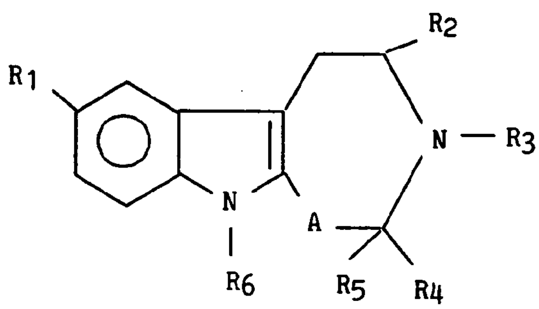

(I)

avec A représentant soit une liaison σ ,les composés de l'invention étant alors les 1,2,3,4- tétrahydro β carbolines de formule générale (II), soit un groupement

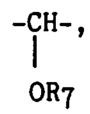

les composés de l'invention étant alors les 1,2,3,4,5,6-hexahydroazépino [4,5-b] indoles de formule générale (III) :

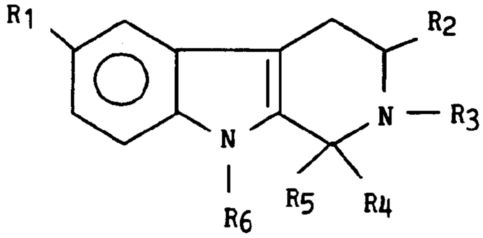

(II)

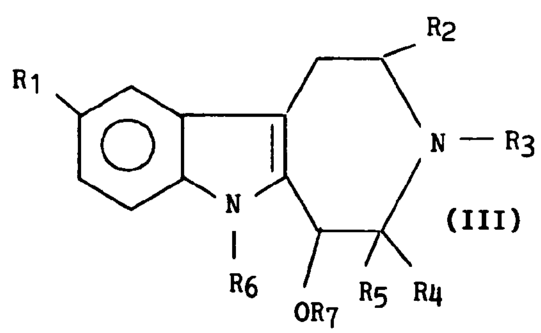

(III)

dans lesquelles :

– $R_1$ représente un atome d'hydrogène ou d'halogène, un groupement hydroxy, alcoxy de 1 à 4 atomes

de carbone linéaire ou ramifié, alkyle inférieur de 1 à 6 atomes de carbone linéaire ou ramifié, éventuellement substitué par un ou des atomes d'halogènes ou groupements oxo, hydroxy, alcoxy de 1 à 4 atomes de carbone,

– $R_2$ représente un groupement carboxy, un groupement alcoxy-carbonyle de 1 à 6 atomes de carbone ramifié ou non éventuellement substitué par un groupement phényle éventuellement substitué, un groupement phényloxycarbonyle éventuellement substitué, un groupement carbamoyle éventuellement substitué sur l'azote par un ou des groupements alkyle inférieur de 1 à 6 atomes de carbone linéaire ou ramifié ou cycloalkyle de 4 à 7 atome de carbone et, dans le cas où A est un groupement de la forme

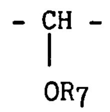 ,

$R_2$ peut également être un atome d'hydrogène,

– $R_3$ représente un atome d'hydrogène, un groupement alkyle inférieur de 1 à 6 atomes de carbone ramifié ou non éventuellement substitué par un ou des atomes d'halogènes, ou groupements oxo, alcoxy inférieur de 1 à 4 atomes de carbone linéaire ou ramifié, phényle éventuellement substitué,

– $R_4$ et $R_5$ forment ensemble un système cyclique mono ou bicyclique saturé ou non, comprenant de 5 à 12 sommets pouvant comprendre dans le squelette cyclique de 0 à 3 hétéroatomes choisis parmi oxygène, azote, soufre et pouvant éventuellement être substitués par un ou des groupements oxo, alkyle inférieur de 1 à 6 atomes de carbone ramifié ou non, phényle éventuellement substitué, phénylcarbonyle éventuellement substitué, phénylalkyle de 7 à 9 atomes de carbone éventuellement substitué, fluorène éventuellement substitué avec, dans le cas où $R_4$ et $R_5$ forment un système bicyclique, la possibilité qu'un des cycles soit un noyau aromatique éventuellement substitué et, dans le cas où A est un groupement

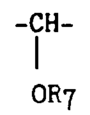 ,

$R_4$ et $R_5$ peuvent également représenter chacun indépendamment l'un de l'autre un atome d'hydrogène, un groupement phényle éventuellement substitué, alkyle inférieur de 1 à 6 atomes de carbone ramifié ou non éventuellement substitué par 1 ou plusieurs groupements oxo, phényle éventuellement substitué, avec la réserve que, lorsque A est une liaison $\sigma$ avec $R_1 = R_3 = R_5 = H$ et $R_2 = COOH$ alors $R_4$ et $R_5$ ne peuvent former ensemble un cyclopentyle ou un cyclohexyle et lorsque A est un groupement

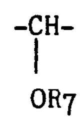

avec $R_7 = H$ ou

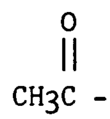 ,

alors $R_4$ et $R_5$ doivent être différents de H,

– $R_6$ représente un atome d'hydrogène, un groupement alkyle inférieur de 1 à 6 atomes de carbone linéaire ou ramifié éventuellement substitué par un ou des groupements oxo, phényle éventuellemnt substitué,

– $R_7$ représente un atome d'hydrogène, un groupement alkyle de 1 à 6 atomes de carbone linéaire ou ramifié éventuellement substitué par un ou des groupements oxo, halogène, alcoxy de 1 à 4 atomes de carbone, cycloalkyle de 3 à 6 atomes de carbone, phényle éventuellement substitué,

– leurs isomères, diastéoisomères, énantiomères,

– leurs sels d'addition à un acide minéral ou organique pharmaceutiquement acceptable ou, dans le cas ou $R_2$ représente un groupement carboxy, leurs sels d'addition à une base minérale ou organique pharmaceutiquement acceptable,

– le terme substitué associé aux expressions phényle, phénylalkyle, phénylcarbonyle signifie que les noyaux aromatiques peuvent être substitués par un ou des groupements alkyle de 1 à 6 atomes de carbone linéaire ou ramifié, alcoxy de 1 à 4 atomes de carbone, hydroxy, nitro, trifluorométhyle ou halogène.

L'invention s'étend également aux procédés d'obtention des composés de formule générale **(I)**. Les procédés d'obtention de ces composés dépendent de la nature du groupement A.

– Soit l'on fait réagir une tryptamine substituée de formule générale **(IV)** :

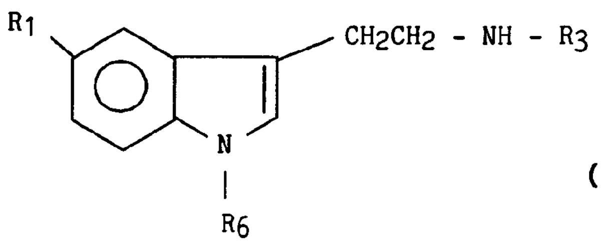

**(IV)**

dans laquelle $R_1$, $R_3$ et $R_6$ ont la même signification que dans le composé de formule générale **(I)** avec un aldéhyde de formule générale **(V)** :

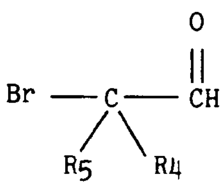

**(V)**

dans laquelle $R_5$ et $R_4$ ont la même signification que dans le composé de formule générale **(I)** en les chauffant dans un acide organique de formule générale **(VI)** :

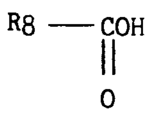

**(VI)**

dans laquelle $R_8$ est un groupement méthyl, éthyl, trifluorométhyl de manière à obtenir le 1,2,3,4,5,6-hexahydroazépino [4,5-b] indole de formule générale **(VII)** :

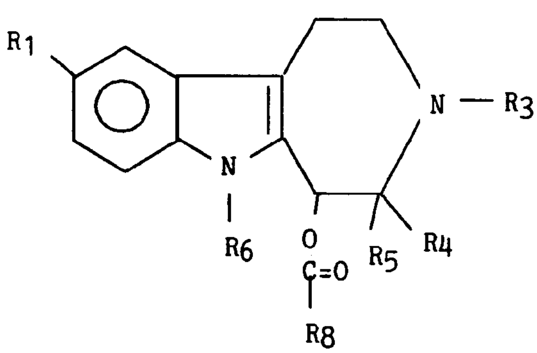

**(VII)**

dans laquelle $R_1$, $R_3$, $R_4$, $R_6$, $R_6$ ont la même signification que dans le composé de formule générale **(I)** et $R_6$

la même signification que dans l'acide **(VI)** que l'on peut, soit chauffer en présence d'un alcool de formule générale $R_9OH$ dans laquelle $R_9$ est un groupement alkyle inférieur de 1 à 6 atomes de carbones ramifié ou non ou un groupement aralkyle de manière à obtenir le 1,2,3,4,5,6-hexahydroazépino [4,5-b] indole de formule générale **(VIII)** :

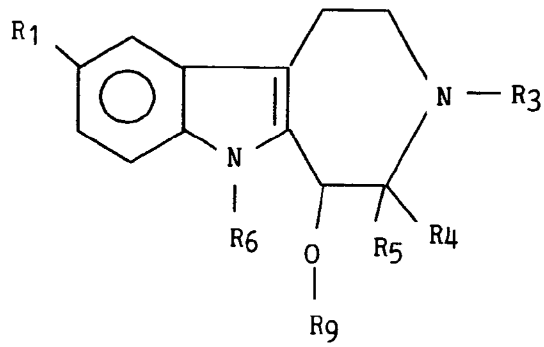

**(VIII)**

dans laquelle $R_1$, $R_3$, $R_4$, $R_5$, $R_5$ ont la même signification que dans le composé de formule générale **(I)** et $R_9$ la même signification que ci-dessus, soit chauffer dans du méthanol en présence de 10 équivalents de carbonate de potassium de manière à obtenir le 1,2,3,4,5,6-hexahydroazépino [4,5-b] indole de formule générale **(IX)** :

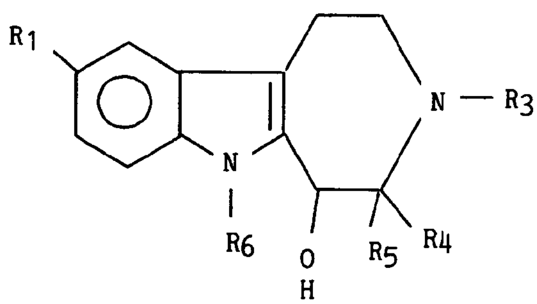

**(IX)**

dans laquelle $R_1$, $R_3$, $R_4$, $R_5$, $R_5$ ont la même signification que dans le composé de formule générale **(I)**,

– soit l'on fait réagir un tryptophane substitué de formule générale **(X)** :

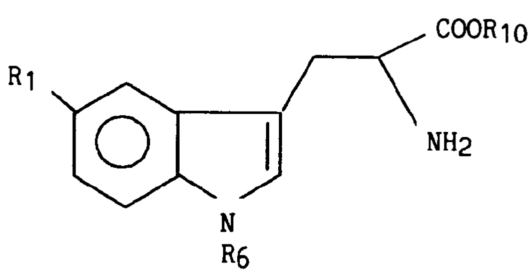

**(X)**

dans laquelle $R_1$, $R_6$ ont la même définition que dans le composé de formule générale **(I)** et $R_{10}$ est un alkyle inférieur de 1 à 6 atomes de carbone ramifié ou non ou un groupement aryle ou aralkyle éventuellement substitué avec une cétone de formule générale **(XI)** :

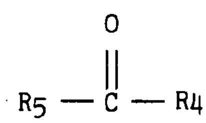

**(XI)**

dans laquelle $R_4$ et $R_5$ ont la même signification que dans le composé de formule générale **(I)** sous atmosphère

d'azote soit dans du toluène ou du benzène au reflux en présence d'acide tosylique, l'eau formée lors de la condensation étant éliminée au moyen d'un appareil extracteur d'eau pouvant être par exemple un Dean Stark, soit, dans certains cas, au reflux du méthanol ou de la cétone elle même, de manière à obtenir après purification la β carboline de formule générale **(XII)** :

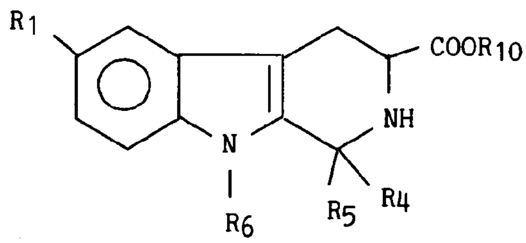

**(XII)**

dans laquelle $R_1$, $R_4$, $R_5$, $R_6$, $R_{10}$ ont la même signification que dans les composés de formule générale **(I)** et **(X)** que l'on peut :

– soit faire réagir dans des conditions d'amination réductrice avec un aldéhyde de formule générale **(XIII)** :

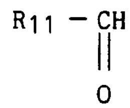

**(XIII)**

dans laquelle $R_{11}$ est un atome d'hydrogène ou un groupement alkyle inférieur de 1 à 6 atomes de carbone ramifié ou non éventuellement substitué par un ou des atomes d'halogènes ou des groupements alcoxy ou aryle substitués ou non de manière à obtenir la β carboline de formule générale **(XIV)** :

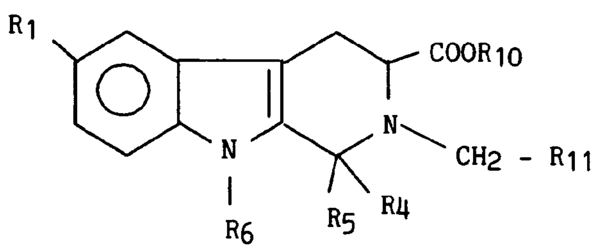

**(XIV)**

dans laquelle $R_1$, $R_{10}$, $R_{11}$, $R_4$, $R_6$, $R_6$ ont la même signification que dans les composés de formule générale **(XII)** et **(XIII),**

– soit faire réagir avec un chlorure d'acide, ou l'anhydride correspondant, de formule générale **(XV)**:

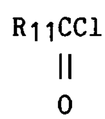

**(XV)**

dans laquelle $R_{11}$ a la même signification que dans le composé de formule générale **(XIII)** de manière à obtenir la β carboline de formule générale **(XVI)** :

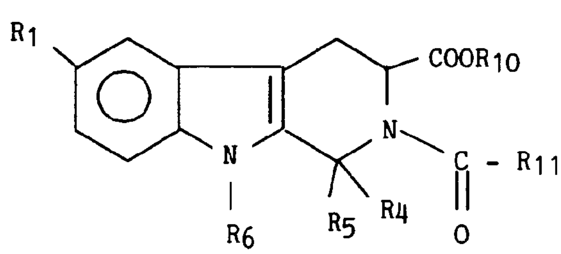

**(XVI)**

dans laquelle $R_1$, $R_{10}$, $R_{11}$, $R_6$, $R_5$, $R_4$ ont la même signification que dans le composé de formule générale **(XIV)**,

– soit faire réagir, dans le cas où $R_{10} = CH_3$, avec une solution aqueuse d'hydroxyde de Baryum de manière à obtenir les acides de formule générale **(XVII)** :

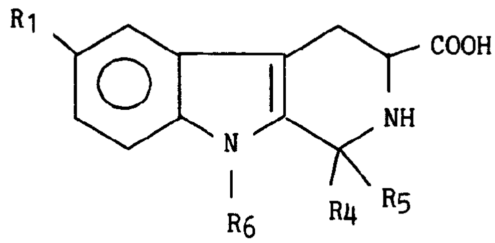

**(XVII)**

dans laquelle $R_1$, $R_4$, $R_5$ et $R_6$ ont la même signification que dans les composés de formule générale **(I)**,

– soit faire réagir dans le cas où $R_{10} = CH_3$, avec une amine de formule générale **(XVIII)** :

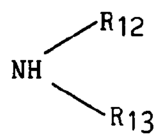

**(XVIII)**

dans laquelle $R_{12}$ et $R_{13}$ représentent chacun indépendamment l'un de l'autre un atome d'hydrogène ou des groupements alkyles inférieurs de 1 à 6 atomes de carbone ramifié ou non ou cycloalkyle de 4 à 7 atomes de carbone, de manière à obtenir les amides de formule générale **(XIX)** :

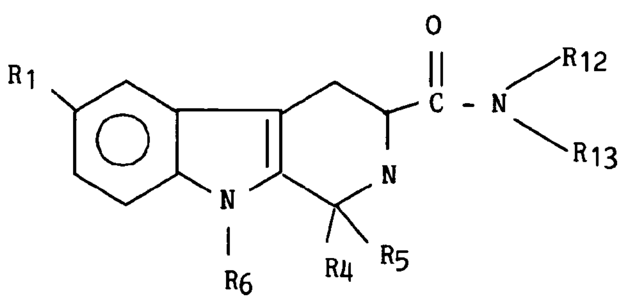

**(XIX)**

dans laquelle $R_1$, $R_4$, $R_5$, $R_5$ ont la même signification que dans les composés de formule générale **(I)** et $R_{12}$ et $R_{13}$ la même signification que dans les amides de formule générale **(XVIII)**,

étant entendu que les composés de formule générale VII, VIII, IX, XII, XIV, XVI, XVII, XIX font partie de l'invention et sont des cas particuliers des composés de formule générale **(I)**.

Les 1,2,3,4,5,6-hexahydroazépino [4,5-b] indoles et 1,2,3,4,-tetrahydro β carbolines de formule générale **(I)** ainsi que leurs sels d'addition à un acide minéral ou organique pharmaceutiquement acceptable ou, lorsque cela est possible, leurs sels d'addition à une base minérale ou organique pharmaceutiquement acceptable sont des principes actifs très intéressants pouvant être utilisés par exemple comme anxiolytiques, antidépresseurs, antipsychotiques, analgésiques. Certains de ces produits sont également anticonvulsivants, myorelaxants, antiinflammatoires et efficaces dans le traitement de désordres vasculaires cérébraux et des troubles de la mémoire.

Les composés de formule générale **(I)** ainsi que leurs sels d'addition à un acide minéral ou organique pharmaceutiquement acceptable comme par exemple les acides chlorhydrique, méthanesulfonique, citrique, maléique ou, si cela est possible, leurs sels d'addition à une base minérale ou organique pharmaceutiquement acceptable comme par exemple les hydroxydes de sodium, de potassium, de calcium, l'arginine, l'éthanolamine ou la diéthanolamine peuvent être élaborés en préparations pharmaceutiques selon des procédés généralement connus comme par exemple en comprimés, gelules, dragées, solutions buvables, solutions injectables, suspensions buvables, émulsions, suppositoires.

Outre les excipients inertes, non toxiques et pharmaceutiquement acceptables tels par exemple l'eau distillée, le glucose, le lactose, l'amidon, le talc, les huiles végétales, l'éthylène glycol etc..., ces préparations peuvent également contenir des agents de préservation : stabilisants, mouillants, émulsifiants etc...

Les compositions ainsi obtenues se présentent généralement sous formes dosées et peuvent contenir selon les affections traitées, l'âge et le poids du malade de 0,1 à 500 mg de principe actif.

Elles peuvent selon le cas être administrées par voie orale, rectale ou parentérale à la dose de 0,1 à 500 mg de une à plusieurs fois par jour.

Les exemples suivants illustrent l'invention et ne la limitent en aucune manière.

**EXEMLE 1 : [ 3-METHOXYCARBONYLE 1,2,3,4-TETRAHYDRO-β-CARBOLINE (3 S)]-1-SPIRO-3′-[ 2-OXO 2,3-DIHYLROINDOLE]**

2,93 g (14,3 mmoles) d'ester méthylique du L (-) tryptophane, 4,2 g (28,6 mmoles) d'isatine et 2,7 g d'acide tosylique (monohydrate) sont dissous à chaud dans la quantité minimale de méthanol permettant d'être en solution à chaud.

Le milieu réactionnel est porté au reflux sous atmosphère d'azote pendant 48 heures puis le méthanol est éliminé par distillation sous pression réduite.

Le résidu obtenu est purifié par chromatographie sur colonne de silice (éluant chloroforme/ méthanol 95/5). Le produit obtenu est ensuite recristallisé dans le méthanol.

On obtient ainsi 2,55 g (55 %) de [ 3-méthoxycarbonyle 1,2,3,4-tétrahydro-β-carboline (3 S)]-1-spiro-3′-[ 2-oxo 2,3-dihydroindole]

Point de fusion : 253° C

Pouvoir rotatoire : $\alpha_D$ (MeOH) : - 151,6°

UV (MeOH) : λ max à 215, 225, 245, 282 et 285 nm

IR (KBr) : νC = 0 à 1715, 1730 , 1620 $cm^{-1}$ autres bandes 3290 et 3370 $cm^{-1}$

RMN $^1H$ 300 MHz ($CDCl_3$)

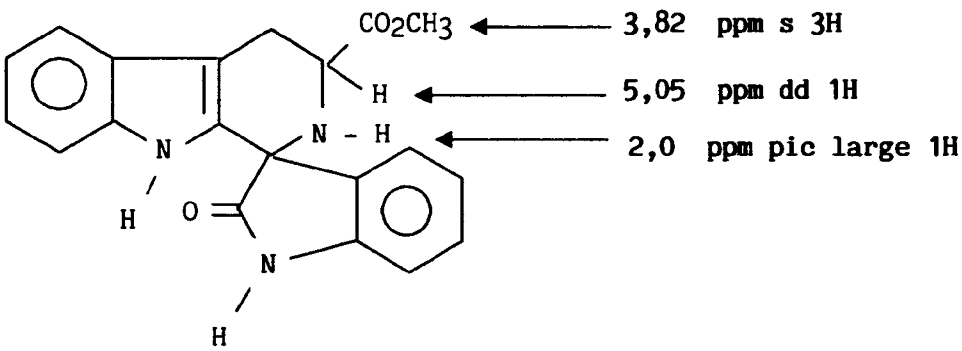


RMN $^{13}C$ 75 MHz ($CDCl_3$)

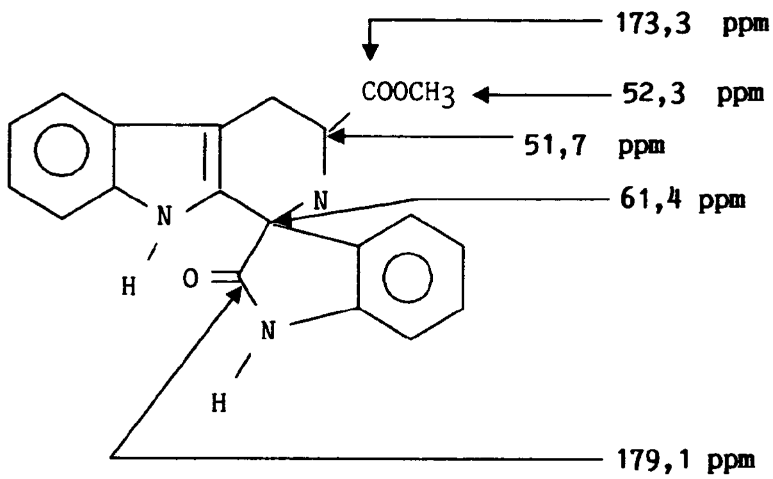

**EXEMPLE 2 : [ 3-METHOXYCARBONYLE 1,2,3,4-TETRAHYDRO-β-CARBOLINE (3 S)]-1-SPIRO-CYCLOHEXANE**

3 g (14,6 mmoles) d'ester méthylique du L (-) tryptophane, 2,86 g (29,2 mmoles) de cyclohexanone et 0,28 g d'acide tosylique (monohydrate) sont dissous à chaud dans suffisamment de toluène pour être en solution au reflux sous atmosphère d'azote dans un appareil de Dean - Stark permettant d'éliminer l'eau formée pendant la réaction.

Le milieu réactionnel est porté au reflux jusqu'à disparition des matières premières (environ 20 heures) puis le toluène est éliminé par distillation sous pression réduite.

Le produit brut obtenu est recristallisé dans le méthanol.

On obtient ainsi 3,7 g (89 %) de [ 3-méthoxycarbonyle 1,2,3,4-tetrahydro-β-carboline (3 S)]-1-spiro-cyclohexane

Point de fusion : 175° C

Pouvoir rotatoire : $\alpha_D$ (MeOH) : - 70,9°

UV (MeOH) : λ max à 225, 280, 285 nm

RMN $^1H$ 300 MHz ($CDCl_3$)

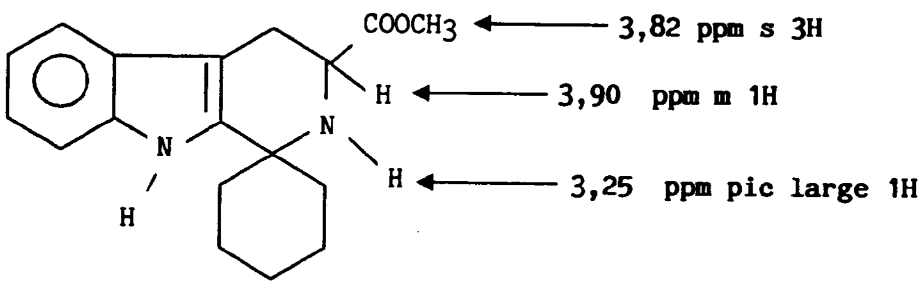


RMN $^{13}C$ 75 MHz ($CDCl_3$)

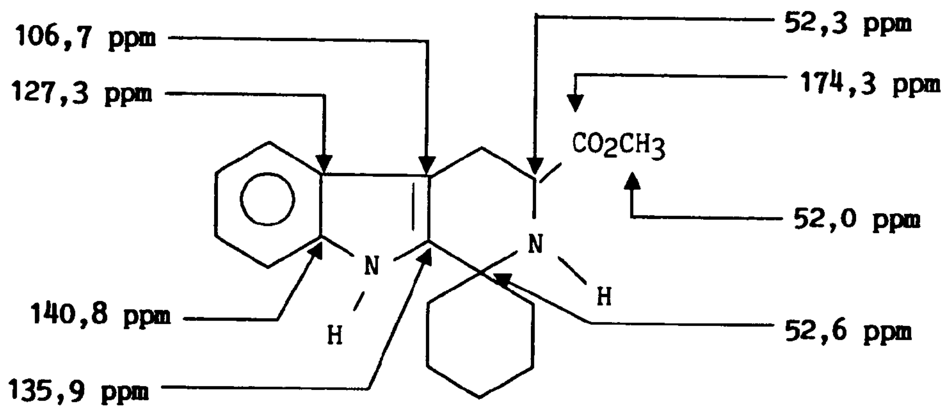


**EXEMPLE 3 : [ 3-CARBOXY 1,2,3,4-TETRAHYDRO-β-CARBOLINE (3 S)]-1-SPIRO - CYCLOHEXANE**

Ajouter 100 cm³ d'une solution aqueuse saturée en baryte à une solution de 1,5 g de [ 3-methoxycarbonyle 1,2,3,4-tétrahydro-β-carboline (3 S)]-1-spiro-cyclohexane dans 100 cm³ de dioxane. Porter au reflux pendant 2 h 30 puis refroidir et faire barbotter du $CO_2$ gazeux jusqu'à fin de précipitation du carbonate de baryum. Eliminer le précipité par filtration puis mettre à sec.

On obtient 0,9 g (63 %) de [ 3-carboxy 1,2,3,4-tetrahydro-β-carboline (3 S)]-1-spiro-cyclohexane

Point de fusion : 198 - 201° C

Pouvoir rotatoire : $\alpha_D$ = - 69,9° (c = 4,81 $gl^{-1}$ MeOH)

UV (MeOH) : λ max 290, 280, 220 nm

RMN $^1H$ ($CDCl_3$ + $CD_3OD$) δ : (ppm)

7,45 d(1H); 7,35 d(1H); 7,15t(1H); 7,05 t(1H); 4,10 dd(1H);

3,35 dd (1H); 3,22 dd(1H); 2,4 à 1,8 massif (10H)

**EXEMPLE 4 : [ 3-METHOXYCARBONYL 1,2,3,4-TETRAHYDRO-β-CARBOLINE (3 S)]-1-SPIRO - CYCLOHEPTANE.**

En procédant comme pour l'exemple 2 mais en remplaçant la cyclohexanone par la cycloheptanone on obtient le [ 3-méthoxycarbonyl 1,2,3,4-tetrahydro-β-carboline (3 s)]-1-spiro - cycloheptane.
Point de fusion : 130 - 133° C
Pouvoir rotatoire : $\alpha_D$ = - 55° (c = 6,00 $gl^{-1}$ MeOH)
UV (MeOH) : λ max 288, 280, 225 nm
IR (film $CHCl_3$) bandes à 3400, 1730 $cm^{-1}$
RMN $^1H$ 300 MHz ($CDCl_3$)

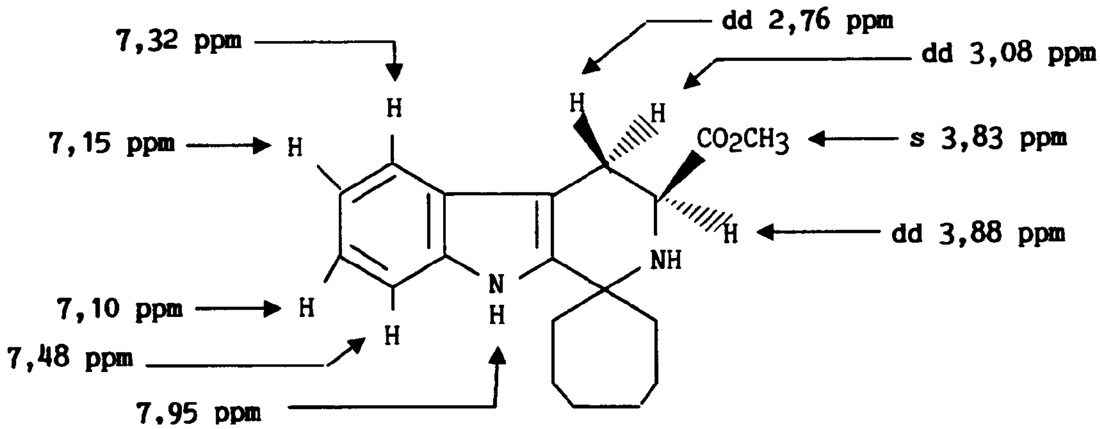


RMN $^{13}C$ 75 MHz ($CDCl_3$)

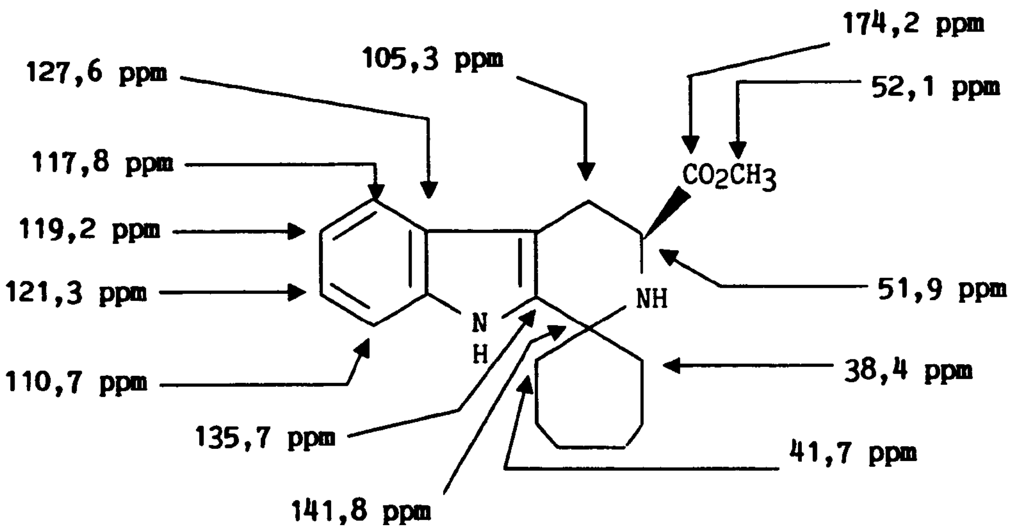


Autres signaux ($CH_2$) à 30,0; 29,6; 26,2; 23,1 et 22,4 ppm

**EXEMPLE 5 : [ 3-(N-PROPYLCARBAMOYL) 1,2,3,4-TETRAHYDRO-β-CARBOLINE (3 S)]-1-SPIRO-CYCLOHEXANE**

Charger en tube scellé 2 g de [ 3-méthoxycarbonyl 1,2,3,4-tétrahydro-β-carboline (3 S)]-1-spiro-cyclohexane et 30 $cm^3$ de N-propylamine puis chauffer la suspension à 60° C pendant 45 heures.

Eliminer la N-propylamine sous pression réduite puis purifier le résidu par chromatographie.

On obtient le [ 3-(N-propylcarbamoyl) 1,2,3,4-tétrahydro-β-carboline (3 S)]-1-spiro-cyclohexane avec un rendement de 50 %
Point de fusion : 225 - 226° C
Pouvoir rotatoire : $\alpha_D$ = - 101,3° (c = 5,77 $gl^{-1}$ MeOH)

UV (MeOH) : λ max 290, 283, 225 nm
IR (film $CHCl_3$) : bandes à 3200 (large), 1650 $cm^{-1}$
RMN $^1H$ 300 MHz ($CDCl_3$)

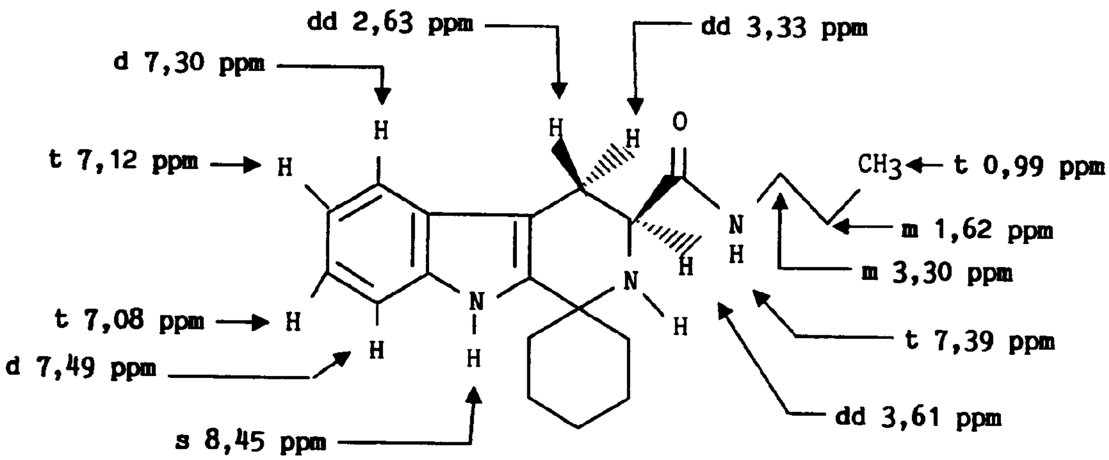


RMN $^{13}C$ 75 MHz ($CDCl_3$)

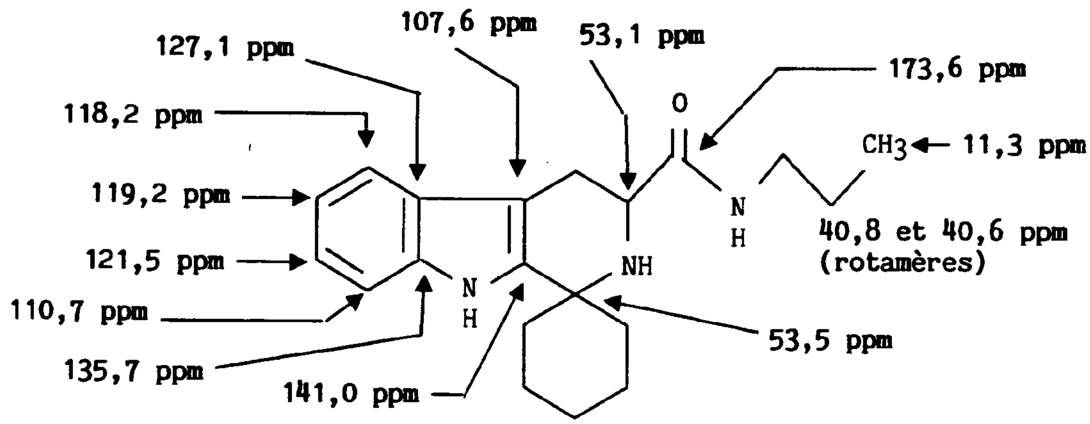


Autres signaux (CH2) à 38,5; 34,3; 25,8; 25,4; 22,8; 21,6 et 21,2 ppm

**EXEMPLE 6 : [3-(N-CYCLOHEXYL CARBAMOYL) 1,2,3,4-TETRAHYDRO-β-CARBOLINE (3 S)]-1-SPIRO-CYCLOHEXANE.**

Chauffer à reflux pendant 20 heures une suspension sous atmosphère d'argon de 2 g de [3-méthoxycarbonyl 1,2,3,4-tetrahydro-β-carboline (3 S)]-1-spiro-cyclohexane dans 35 $cm^3$ de cyclohexylamine.

Eliminer la cyclohexylamine par distillation puis purifier le résidu par chromatographie.

On obtient le [3-(N-cyclohexylcarbamoyl) 1,2,3,4-tetrahydro-β-carboline (3 S)]-1-spiro-cyclohexane avec un rendement de 63 %.

Point de fusion : 225 - 230°C
pouvoir rotatoire : $\alpha_D$ = - 60,8° (c = 5,75 $gl^{-1}$ MeOH)
UV(MeOH) : λ max 289, 280, 225 nm
IR (KBr) 3290, 1660, 1580 $cm^{-1}$
RMN $^1H$ 300 MHz ( $CDCl_3$)

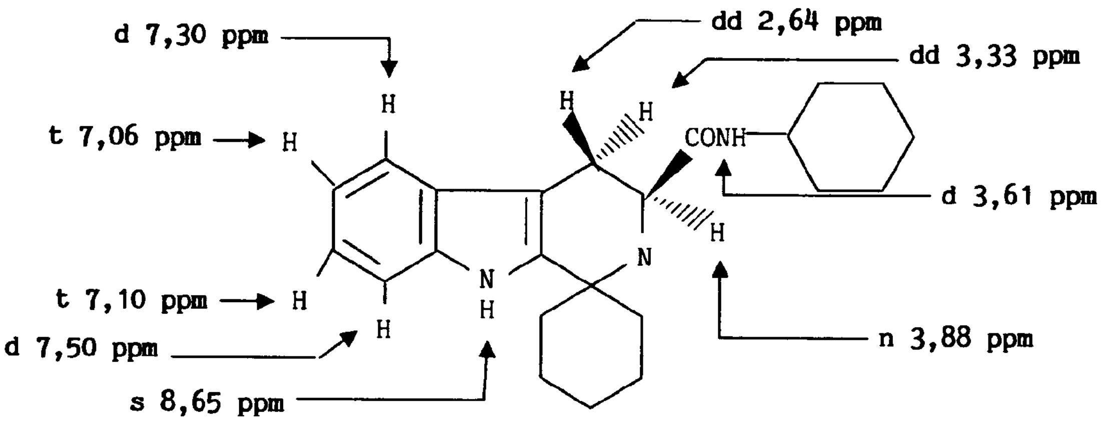


RMN $^{13}C$ 75 MHz ($CDCl_3$)

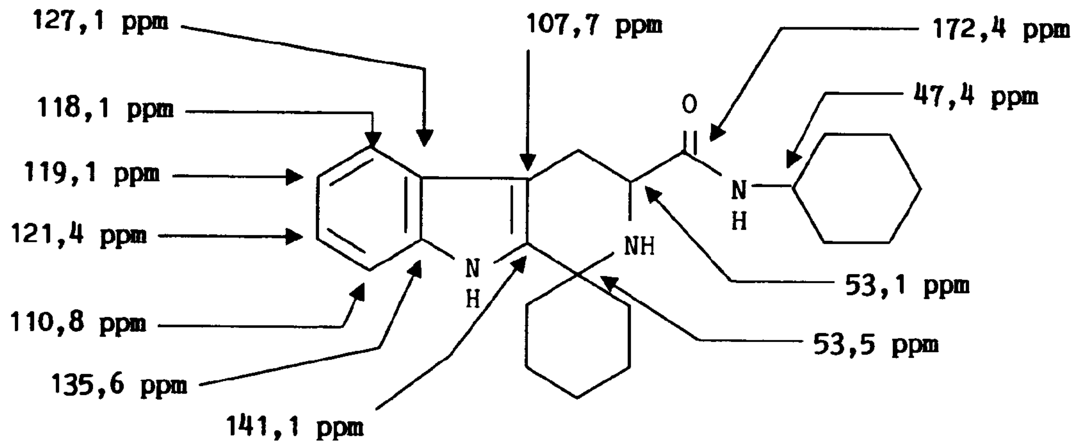


Autres signaux ($CH_2$) à 38,5; 34,1; 33,0; 32,9; 25,8; 25,5; 24,5; 21,7; 21,2 ppm

**EXEMPLE 7 : [ 3-CARBMOYL 1,2,3,4-TETRAHYDRO-β-CARBOLINE (3 S) ]-1-SPIRO-CYCLOHEXANE**

Dissoudre en tube scellé 1 g de [ 3-méthoxycarbonyl 1,2,3,4-tetrahydro-β-carboline]-1-spiro-cyclohexane dans 12 $cm^3$ de méthanol puis ajouter 3 $cm^3$ d'ammoniac liquide et maintenir à température ambiante pendant 36 heures.

Laisser dégazer l'ammoniac et isoler par filtration le produit formé qui cristallise lentement.

On obtient ainsi 0,82 g (82 %) de [ 3-carbamoyl 1,2,3,4-tétrahydro-β-carboline]-1-spiro-cyclohexane
pouvoir rotatoire :$\alpha_D$ = - 117,6° (c - 4,02 $gl^{-1}$ DMSO)
Point de fusion base : 278 - 279° C chlorhydrate : 188 - 189° C
UV : λ max 295 et 220 nm
IR (pastille KBr) : bandes à 3500, 3250, 1680, 1560 $cm^{-1}$
RMN $^1H$ (base) ($CDCl_3$ + DMSO $d_6$) δ (ppm)
10,3 sl(1H); 7,4d (1H); 7,3 d(1H); 7,05 t(1H); 6,95 t(1H);
6,8 sl (1H); 4,6 sl(2H); 3,6 m(1H); 3,15 dd(1H); 2,6 dd(1H)
2,15 dt (1H); 1,9 - 1,3 massifs(9H)

**EXEMPLE 8 : [ 3-METHOXYCARBONYLE 1,2,3,4-TETRAHYDRO-β-CARBOLINE (3 S)]-1-SPIRO-4'- [ 1-METHYL PIPERIDINE]**

3,7 g (17 mmoles) d'ester méthylique de L(-) tryptophane, 3,85 g (34 mmoles) de N-méthyl 4-pipéridinone redistillée et 0,34 g d'acide tosylique (monohydrate) sont dissous à chaud, dans suffisamment de toluène pour être en solution au reflux, sous atmosphère d'azote dans un appareil de Dean Stark permettant d'éliminer l'eau formée pendant la réaction.

Le milieu réactionnel est porté au reflux jusqu'à disparition des matières premières (environ 20 heures)

puis le toluène est éliminé par distillation sous pression réduite . Le résidu obtenu est repris par du chloroforme et longuement lavé par une solution aqueuse à 10 % de bicarbonate de sodium de manière à éliminer au maximum l'excès de N-méthyl 4-pipéridinone.

Après mise à sec de sa solution chloroformique, le produit brut est purifié par chromatographie sur colonne de silice (Eluant $CHCl_3/CH_3OH$ avec gradient de concentration)

On obtient ainsi 3,4 g (64 %) de [ 3-méthoxycarbonyl 1,2,3,4-tétrahydro-β-carboline (3 S)]-1-spiro-4'-[ 1-méthyl pipéridine] sous forme de produit non cristallisé.

Pouvoir rotatoire : $\alpha_D$ (MeOH) : - 19,5°

UV (MeOH) : λ max à 220, 280, et 296 nm

IR (film $CHCl_3$) νC = 0 à 1725 et 1740 $cm^{-1}$

RMN $^1H$ 300 MHz ($CDCl_3$)

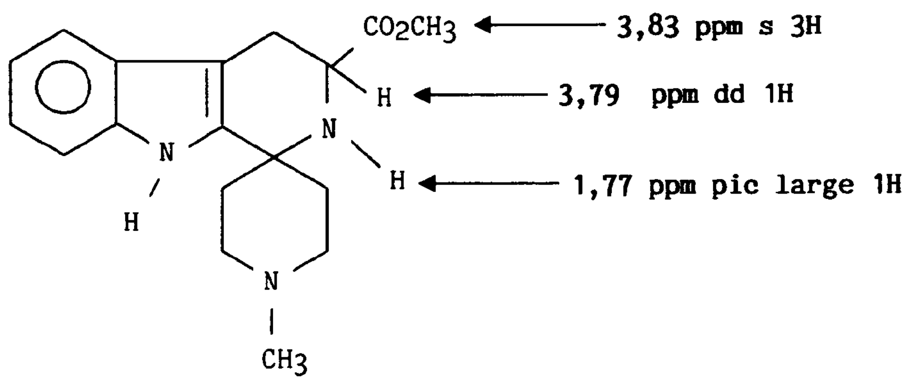


RMN $^{13}C$ 75 MHz ($CDCl_3$)

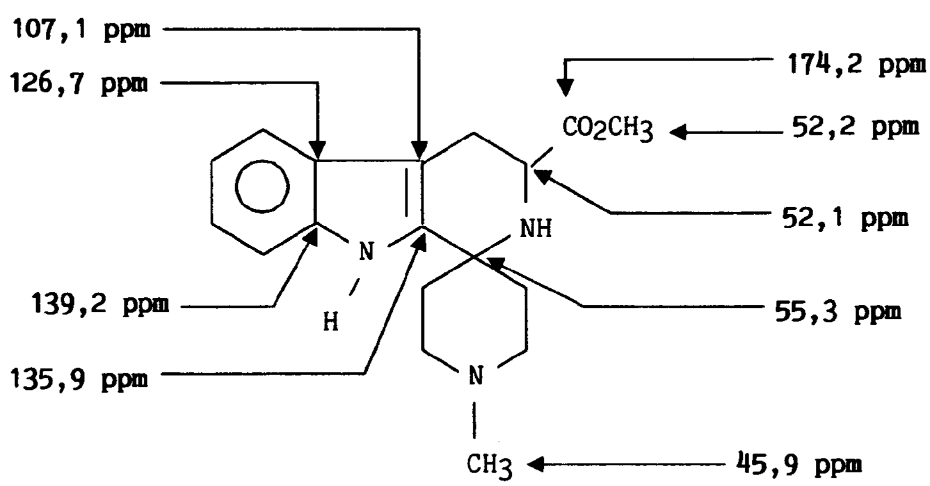


**EXEMPLE 9 : [ 3-CARBOXY 1,2,3,4-TETRAHYDRO-β-CARBOLINE (3 S)]-1-SPIRO-4'-[ 1-METHYL PIPERIDINE]**

1 g (31,9 mmoles) de [ 3-méthoxycarbonyl 1,2,3,4- β carboline(3 S)]-1-spiro-4'-[1-méthyl pipéridine] est ajouté à un mélange de 20 $cm^3$ d'eau distillée et 2 $cm^3$ de lessive de potasse à 38 %.

On ajoute la quantité de dioxane permettant d'obtenir la dissolution à chaud et le milieu réactionnel est maintenu 10 heures au reflux sous atmosphère d'azote. Le milieu réactionnel est ensuite concentré aux 2/3 et l'on ajoute 100 mg de résine cationique Amberlist 77. La résine est éliminée par filtration et le milieu réactionnel concentré sous pression réduite.

On obtient ainsi 0,7 g (67 %) de [ 3-carboxy 1,2,3,4-tétrahydro-β-carboline(3S)]-1-spiro-4'-[ 1-méthyl pipéridine]

UV (MeOH) : λ max à 220, 280, et 290 nm

IR (film ) targe bande vers 1700 $cm^{-1}$

**EXEMPLE 10 : [ 3-METHOXYCARBONYL 2-METHYL 1,2,3,4-TETRAHYDRO-β-CARBOLINE (3 S)]-1-SPIRO-4′-[ 1-METHYL PIPERIDINE]**

98 mg (0,31 mmoles) de [ 3-méthoxycarbonyl 1,2,3,4-tétrahydro-β-carboline(3S)]-1-spiro-4′-[ 1-méthyl pipéridine] et 39 mg de cyanoborohydrure de sodium sont dissous dans un mélange de 4 cm³ de formol à 40 % et 0,5 cm³ d'acide acétique. Après une heure d'agitation à température ambiante le milieu réactionnel est versé dans une solution aqueuse à 10 % de bicarbonate de sodium et extrait au chloroforme. Les phases chloroformiques sont lavées à l'eau, séchées sur sulfate de magnésium puis mise à sec sous pression réduite.

On obtient ainsi 95 mg (93 %) de [ 3-méthoxycarbonyl 2-méthyl 1,2,3,4-tetrahydro-β-carboline(35)]-1-spiro-4′-[ 1-méthyl pipéridine] sous forme de gommes.

Pouvoir rotatoire : $\alpha_D$ (MeOH) : - 8°

UV (MeOH) : λ max à 225, 280, 285 nm

IR (film $CHCl_3$) νC = 0 à 1720 et 1735 $cm^{-1}$

RMN $^1H$ 300 MHz ($CDCl_3$)

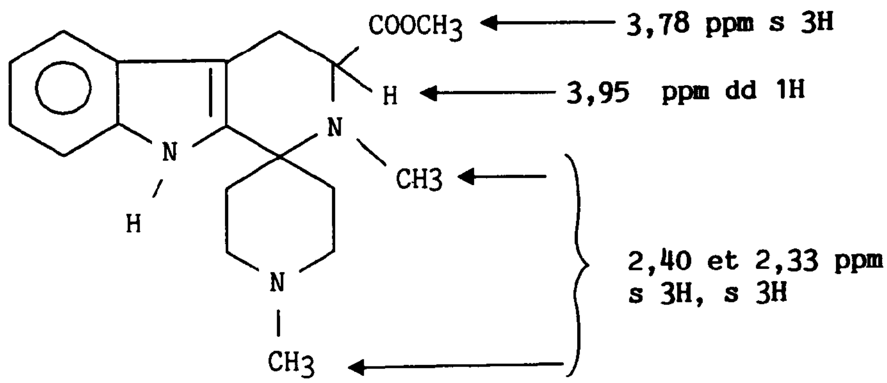


RMN $^{13}C$ 75 MHz ($CDCl_3$)

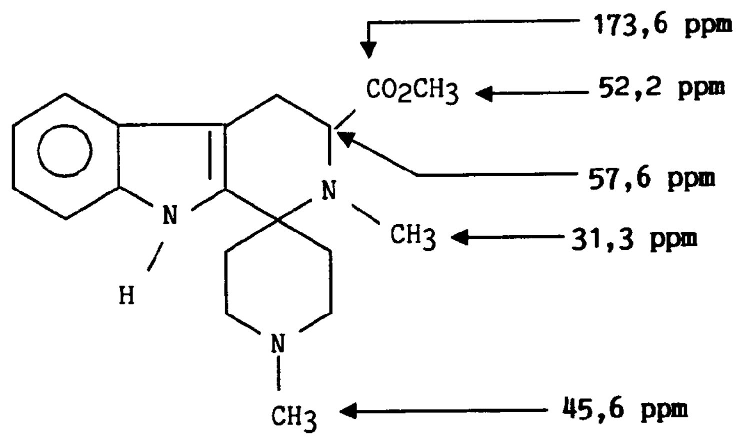


**EXEMPLE 11 : [ 3-CARBOXY 1,2,3,4-TETRAHYRO-β-CARBOLINE (3S) ]-1-SPIRO-4′-PIPERIDINE**

Chauffer au reflux pendant 48 heures sous atmosphère d'argon un mélange de 2,5 g de tryptophane et 1,8 g de chlorhydrate de pipéridin-4-one en solution dans un mélange de 70 cm³ de dioxane, 40 cm³ d'eau et 5 cm3 d'acide sulfurique à 97 %.

Refroidir le milieu réactionnel et isoler par filtration le produit formé qui précipite lentement.

On obtient ainsi 2,3 g (62 %) de chlorhydrate de [ 3-carboxy 1,2,3,4-tetrahydro-β-carboline (35)]-1-spiro-4′-piperidine.

UV (MeOH) : λ max 295 et 222 nm

RMN $^1H$ ($CDCl_3$ + $CD_3OD$) δ (ppm)

7,6 d(1H); 7,4 d(1H); 7,15 t(1H); 7,05 t(1H); 4,2 t(1H); 3,9 multiplet (2-3H); 3,5 multiplet (4H); 2,9 multiplet (3-4H)

RMN $^{13}C$ ($CD_3OD$) δ (ppm)
171,6; 137,5; 130,0; 128,3; 123,5; 120,7; 119,2; 112,4; 105,7;
58,3; 54,5; 43,1; 42,0; 27,2; 25,9

**EXEMPLE 12 : [ 3-METHOXYCARBONYL 1,2,3,4-TETRAHYDRO-β-CARBOLINE (3S) ]-1-SPIRO-4′-PIPERIDINE**

Chauffer au reflux pendant 29 heures dans un appareil de Dean Stark une suspension de 2,18 g de tryptophanate de méthyl et 1,55 g de chlorhydrate d'hydrate de 4-pipéridinone dans 160 cm³ de benzène.

Eliminer le benzène par distillation, dissoudre le résidu dans suffisamment de méthanol et faire barbotter de l'HCl gazeux jusqu'à saturation.

Le produit qui cristallise lentement est isolé par filtration.

On obtient ainsi 2,15 g (58 %) de dichlorhydrate de [ 3-méthoxycarbonyl 1,2,3,4-tetrahydro-β-carboline(35)]-1-spiro-4′-pipéridine.

Point de fusion : 182 - 185° C
Pouvoir rotatoire : $\alpha_D$ = - 46,8° (c = 2,71 $gl^{-1}$ $CHCl_3$ - $CH_3OH$ 3 - 1)
UV (MeOH) : λ max 295 et 220 nm
RMN $^1H$ (DMSO $d_6$) δ (ppm) produit sous forme de dichlorhydrate
11,5 s(1H); 9,8 s(1H); 9,4 s(1H); 7,5 d, J = 7Hz(1H);
7,45 d, J = 7Hz (1H); 7,15 t J = 7Hz (1H); 7,05 t J = 7Hz (1H);
4,65 m (1H); 3,9 s(3H); 3,4 m (2H); 3,2-2,4 massifs 8H
RMN $^{13}C$ (DMSO $d_6$) δ (ppm) Produit sous forme de dichlorhydrate.

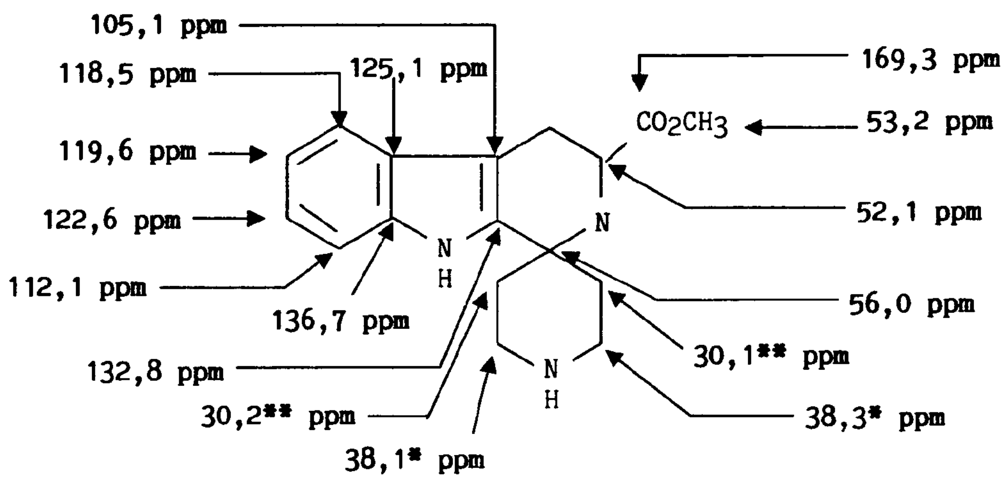


**EXEMPLE 13 : [ 5-ACETOXY 1,2,3,4,5,6-HEXAHYDROAZEPINO [4,5-b] INDOLE ]-4-SPIRO-1′-(CYCLOEXENE-3′)**

1,96 g (10,37 mmoles) de 4-bromo 4-formyl cyclohexène sont ajoutés à une solution de 0,833 g (5,2 mmoles) de tryptamine dans 50 cm³ d'acide acétique puis le milieu réactionnel est chauffé à 70°C sous atmosphère d'azote pendant 15 heures.

Après élimination de l'acide acétique par distillation sous pression réduite, le résidu est repris par 100 cm³ de chlorure de méthylène. La solution organique est lavée par 100 cm³ d'une solution aqueuse à 10 % de carbonate de sodium, séchée sur sulfate de magnésium puis mise à sec ce qui fournit 2,52 g de produit brut.

Une purification par chromatographie sur colonne de silice (Eluant $CHCl_3$) permet d'isoler 1,12 g (70 %) de [5-acétoxy 1,2,3,4,5,6-hexahydroazépino [ 4,5-b] indole ]-4-spiro-1′-(cyclohexène-3′) sous forme d'un mélange de deux diastéroisomères dont la proportion 60/40 a été déterminée par RMN.

UV (MeOH) : λ max à 220, 280, 290 nm
IR (KBr) νC = 0 : 1725 $cm^{-1}$ νC - 0 - C : 1245, 1235 $cm^{-1}$
RMN $^1H$ 300 MHz ($CD_3OD$) δ (ppm)

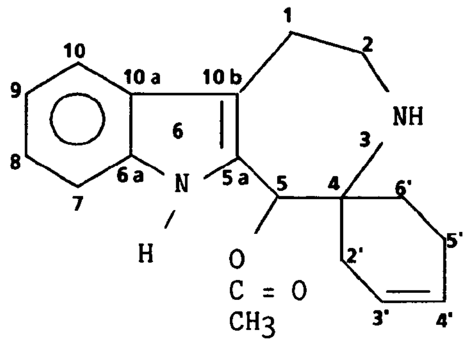

| | | | | |
|---|---|---|---|---|
| 7,55 | 1H | d | J = 8Hz | C(10)H |
| 7,35 | 1H | d | J = 8Hz | C(7)H |
| 7,18 | 1H | t | J = 8Hz | C(8)H |
| 7,1 | 1H | t | J = 8Hz | C(9)H |
| 5,85 et 5,71 | 1H | s (3:2) | | C(5) - H |
| 5,83 - 5,73 | 1H | m | | C(3')H ou C(4')H |
| 5,66 - 5,5 | 1H | m | | C(4')H ou C(3')H |
| 3,33 - 3,14 | 2H | m | | C(2) - HH' |
| 3,14 - 2,96 | 2H | m | | C(1) - HH' |
| 2,33 - 1,63 | 6H | m | | |
| 2,1 et 2,0 | 3H | s (3 : 2) | | $OCOCH_3$ |

RMN $^{13}C$ 75 MHz ($CD_3OD$) δ (ppm)

171,3 ($O\underline{C}OCH_3$) ; 135,0 (C 6a) ; 131,2 (C 5a) ; 127,6 (C 10a) ; 126,5 et 126,0 (C 3′) ; 123,3 (C4′) ; 122,1 et 121,7 (C 8) ; 119,4 et 119,2 ( C 9) ; 118,3 et 118,0 (C 10) ; 113,3 ( C 10b) ; 111,3 (C 7); 75,1 et 71,9 (C 5) ; 55,0 (C 4) ; 41,9 et 41,8 (C 2) ; 36,0 et 33,6 (C 2′) ; 31,7 et 31,4 ; 26,1 ; 22,7 ; 20,7 ($OCO\underline{CH_3}$)

**EXEMLE 14 : [ 5-METHOXY 1,2,3,4,5,6-HEXAHYDROAZEPINO [4,5-b] INDOLE ]-4-SPIRO-1′- (CYCLOHEXENE-3′)**

Un simple chauffage dans le méthanol au reflux pendant 1 heure permet la transformation du [5-acétoxy 1,2,3,4,5,6 - hexahydroazépino [4,5-b]indole]-4-spiro-1′- (cyclohexène-3′) en dérivé 5-méthoxylé.
UV (MeOH) : λ max à 220, 280, 290 nm
IR (film) 3380, 3010, 2920, 1460, 1450, 1090 et 900 $cm^{-1}$
RMN $^1H$ 300 MHz ( $CD_3OD$ ) δ ( ppm)

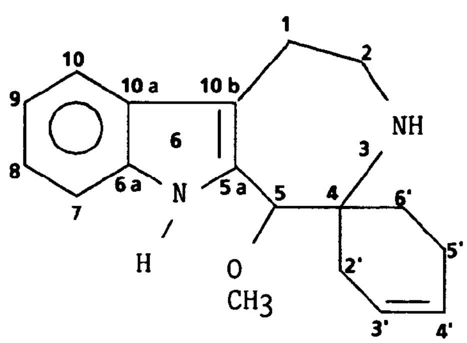

| | | | |
|---|---|---|---|
| 8,20 et 8,15 | 1H | sl (3 : 2) | N(6)H |
| 7,52 | 1H | d J = 8Hz | C(10) -H |
| 7,34 | 1H | d J = 8Hz | C(7) -H |
| 7,22 - 7,06 | 2H | m | C(8) -H + C(9) -H |
| 5,76 - 5,47 | 2H | m | C(3')H + C(4') -H |
| 4,18 et 4,00 | 1H | s (3 : 2) | C(5) -H |
| 3,34 et 3,27 | 3H | s (3 : 2) | $OCH_3$ |
| 3,22 - 2,82 | 4H | m | C(1)-HH' + C(2)-HH' |
| 2,75 - 2,48 | 1H | m | N (3) H |
| 2,34 - 1,54 | 6H | m | |

**EXEMPLE 15 : [ 9-METOXY 5-ACETOXY 1,2,3,4,5,6-HEXAHYDROAZEPINO [ 4,5-b] INDOLE ]-4-SPIRO-1'- (CYCLOEXENE-3')**

En partant de la 5-méthoxy tryptamine et en procédant comme pour l'exemple 6 on obtient le ( 9-méthoxy 5-acétoxy 1,2,3,4,5,6-hexahydroazépino [4,5-b]indole]-4-spiro-1'-(cyclohexène-3') sous forme d'un mélange de diastéroisomères.

UV (MeOH) : λ max à 220, 280, 290 nm

IR (film) νC = 0 : 1710 $cm^{-1}$ νC - 0 - C : 1235, 1215 $cm^{-1}$

RMN $^1H$ 300 MHz ($CDCl_3$) δ (ppm)

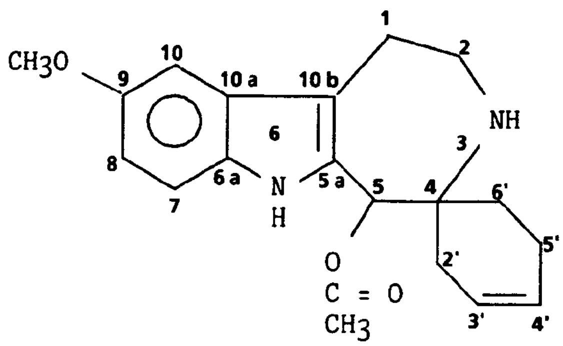

| | | | |
|---|---|---|---|
| 8,46 | 1H | sl | N(6)H |
| 7,16 | 1H | d | C(7) -H |
| 6,94 | 1H | s | C(10) -H |
| 6,84 | 1H | d | C(8) -H |
| 5,72 | 1H | s | C(5) -H |
| 5,82 - 5,66 | 1H | m | C(3')-H ou C(4')H |
| 5,60 - 5,49 | 1H | m | C(4')-H ou C(3')-H |
| 3,85 | 3H | s | $OCH_3$ |
| 3,29 - 3,16 | 2H | m | C(2)HH' |
| 3,02 - 2,87 | 2H | m | C(1)HH' |
| 2,67 - 2,52 | 1H | m | N(3) -H |
| 2,33 - 1,64 | 6H | m | |
| 2,07 | 3H | s | 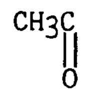 |

**EXEMPLE 16 : [5-ACETOXY 3-METHYL 1,2,3,4,5,6-HEXAHYDROAZEPINO [ 4,5-b] INDOLE]-4-SPIRO-1′- (CYCLOEXENE-3′)**

Une solution de 0,2 g (1,15 mmoles) de N-méthyl tryptamine et 0,240 mg (1,27 mmoles) de 4-bromo 4-formyl cyclohexène dans 20 $cm^3$ d'acide acétique est chauffée 4 jours à 70° C sous atmosphère d'azote. Après élimination de l'acide acétique par distillation sous pression réduite, le résidu est repris par 50 $cm^3$ de chlorure de méthylène et lavé par une solution aqueuse à 10 % de carbonate de sodium. La phase organique est séchée sur $MgSO_4$ filtrée et concentrée sous pression réduite. Le produit brut obtenu est purifié par chromatographie sur silice (Eluant $CH_2Cl_2$ / $CH_3OH$ 97 % / 3 %)

On obtient finalement le [5-acétoxy 3-méthyl 1,2,3,4,5,6-hexahydroazépino [ 4,5-b] indole ]-4-spiro-1′-(cyclohexène-3′) avec un rendement de 42 % sous forme d'un mélange de diastéroisomères.

UV (MeOH) : λ max à 223, 285, 292 nm

IR (film) 3380, 3000, 2900, 1710, 1450, 1360, 1330, 1230, 1010, 950, 710 $cm^{-1}$

RMN $^1H$ 300 MHz ($CDCl_3$) δ (ppm)

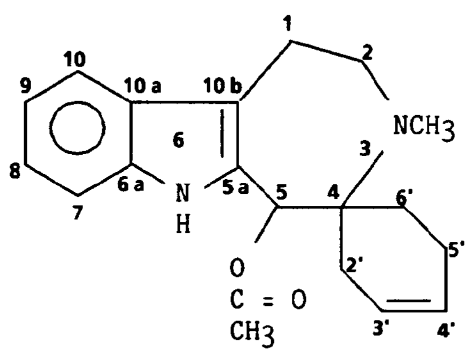

| | | | | |
|---|---|---|---|---|
| 8,38 et 8,35 | 1H | sl | | N(6) -H |
| 7,53 | 1H | d | J = 8Hz | C(10) -H |
| 7,27 | 1H | d | J = 8Hz | C(7) -H |
| 7,19 | 1H | t | J = 8Hz | C(8) -H |
| 7,10 | 1H | t | J = 8Hz | C(9) -H |
| 5,97 et 5,95 | 1H | s (2 : 3) | | C(5) -H |
| 5,79 - 5,66 | 1H | m | | C(3') -H ou C(4') -H |
| 5,64 - 5,47 | 1H | m | | C(4') -H ou C(3') -H |
| 3,68 - 3,54 | 1H | m | | C(2) -H |
| 3,46 - 3,07 | 3H | m | | C(2) -H' + C(1) -HH' |
| 2,90 - 2,72 | 1H | m | | |
| 2,86 et 2,82 | 3H | s (3 : 2) | | N - $\underline{CH_3}$ |
| 2,45 - 1,80 | 5H | m | | |
| 2,08 et 2,06 | 3H | s (3 : 2) | | 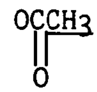 |

**EXEMPLE 17 : 9-METHOXY 5-ACETOXY 4,4-DIMETHYL 1,2,3,4,5,6-HEXAHYDROAZEPINO [4,5-b] INDOLE**

Une solution de 5,34 g de 5-méthoxy tryptamine et 4,53 g de 2-bromo 2-formyl propane dans 150 $cm^3$ d'acide acétique est chauffée à 60° C sous atmosphère d'azote pendant 2 heures. L'acide acétique est éliminé par distillation sous pression réduite et le résidu repris par du chlorure de méthylène et lavé par une solution aqueuse de carbonate de sodium à 10 %. La phase organique est séchée sur $MgSO_4$, filtrée et concentrée sous pression réduite.

On obtient le 9-méthoxy 5-acétoxy 4,4-diméthyl 1,2,3,4,5,6-hexahydroazépino [4,5-b] indole avec un rendement de 66 %

UV (MeOH) : $\lambda$ max à 215, 285, 300 nm

IR (film) 3380, 2960, 2940, 2840, 1730 ($\nu$C = 0),1480, 1450, 1370, 1250 ( $\nu$C - 0 - C ) ,1020, 960 $cm^{-1}$

RMN $^1H$ 300 MHz ($CDCl_3$) $\delta$(ppm)

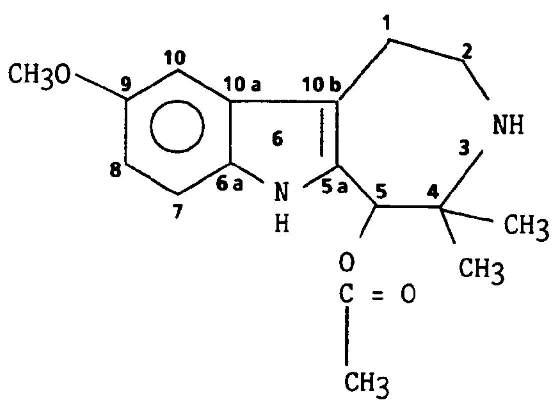

| | | | |
|---|---|---|---|
| 8,55 | 1H | sl | N(6) -H |
| 7,16 | 1H | d | C(7) -H |
| 6,94 | 1H | d | C(10) -H |
| 6,82 | 1H | dd | C(8) -H |
| 3,85 | 3H | s | $\underline{CH_3}$ - O |
| 3,27 - 3,17 | 2H | m | C(1) -HH' |
| 2,96 - 2,85 | 2H | m | C(2) -HH' |
| 2,18 | 1H | sl | N(3)-H |
| 2,08 | 3H | s | 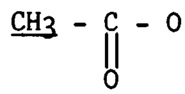 |
| 1,20 et 1,24 | 2X3H | 2Xs | - $CH_3$, - $CH_3$ |

RMN $^{13}C$ 75 MHz ($CDCl_3$) $\delta$ (ppm)
171,1(C = 0), 154,0(9), 132,6 (5a), 130,0 (6a), 128,3 (10a), 113,6 (10b), 112,5 (8), 111,9 (7), 100,2 (10), 77,6 (5), 55,8 ($CH_3O$), 53,9 (4), 42,6 (2), 28,8 (CH3), 27,7 (1), 23,7 (CH3), 21,0

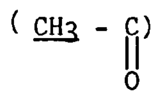

**EXEMPLE 18 : 5-HYDROXY 4,4-DIMETHYL 1,2,3,4,5,6-HEXAHYDROAZEPINO [4,5-b] INDOLE**

STADE 1 : 5-acétoxy 4,4-diméthyl 3-trifluoroacétyl 1,2,3,4,5,6-hexahydroazépino [4,5-b] indole

Une solution de 3,2 g de tryptamine et 3 g de 2-bromo 2-formyl propane dans 150 $cm^3$ d'acide acétique est chauffée à 60° sous atmosphère d'azote pendant 15 heures. L'acide acétique est éliminée par distillation sous pression réduite, le résidu repris par du chlorure de méthylène et lavé par une solution aqueuse à 10 % de carbonate de sodium. Après mise à sec de la solution organique, le résidu est repris dans 10 $cm^3$ de pyridine et 5 $cm^3$ d'anhydride trifluoroacétique . Le milieu réactionnel est versé dans de l'eau et extrait au chlorure de méthylène puis mis à sec.

Le produit brut est recristallisé dans l'acétone.

On obtient le 5-acétoxy 4,4-dimétyl 3-trifluoroacétyl 1,2,3,4,5,6-hexahydroazépino [4,5-b] indole avec un rendement de 34 %.

STADE 2 : 5-hydroxy 4,4-diméthyl 1,2,3,4,5,6-hexahydroazépino [4,5-b] indole

2 g de 5-acétoxy 4,4-diméthyl 3-trifluoroacétyl 1,2,3,4,5,6-hexahydroazépino [4,5-b] indole et 0,7 g de carbonate de potassium dans 50 cm3 de méthanol sont agités à température ambiante sous atmosphère d'azote pendant 4 heures. Après filtration le produit est extrait au chlorure de méthylène et isolé par mise à sec. Le chlorhydrate de 5-hydroxy 4,4-diméthyl 1,2,3,4,5,6-hexahydroazépino [4,5-b] indole est obtenu par dissolution de la base dans l'acétone et addition d'une solution éthérée d'acide chlorhydrique.

Rendement : 95 %

Analyse de la base :

UV (MeOH) : $\lambda$ max à 220, 283, 290 nm

IR (film) 3380, 2960, 2900, 1450 $cm^{-1}$

RMN $^1H$ 300 MHz ($CDCl_3$) $\delta$ (ppm)

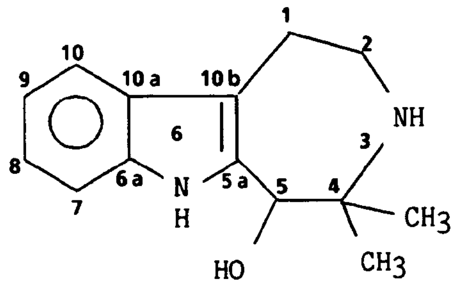

| | | | |
|---|---|---|---|
| 8,56 | 1H | sl | N(6)-H |
| 7,45 | 1H | d | C(10)-H |
| 7,20 | 1H | d | C(7)-H |
| 7,15 et 7,05 | 2H | t,t | C(9)-H et C(8)-H |
| 4,10 | 1H | s | C(5)-H |
| 3,10 - 2,70 | 6H | m | C(1)-HH', C(2)-HH', N(3)-H, OH |
| 1,09 | 3H | s | $CH_3$ |
| 1,01 | 3H | s | $CH_3$ |

**EXEMPLE 19 : COMPRIMES DOSES A 50 MG DE [3-METHOXYCARBONYL 1,2,3,4,-TETRAHYDRO-β-CARBOLINE (3S) ]-1-SPIRO-CYCLOHEXANE**

Formule de préparation pour 1000 comprimés.

[3-méthoxycarbonyl 1,2,3,4,-tétrahydro-β-carboline (3 S) ]-1-spiro-cyclohexane -------------------------------- 50 g
Amidon de blé ------------------------------ 15 g
Amidon de mais ----------------------------- 15 g
Lactose ------------------------------------ 50 g
Stéarate de magnésium ---------------------- 1 g
Silice ------------------------------------- 1 g
Hydroxypropyl cellulose -------------------- 2 g

**ETUDE PHARMACOLOGIQUE DES DERIVES DE L'INVENTION**

**EXEMPLE A : RECHERCHE DE L'ACTIVITE ANTIINFLAMMATOIRE**

Le potentiel antiinflammatoire des composés a été recherché selon un modèle d'inflammation aigue provoquée par injection sous cutanée de carragénine au niveau de la patte postérieure du rat selon une technique inspirée de WINTER et coll. Proc. Soc., Exp. Biol Med, 111, 554 (1962) et WINEGAR et coll. J. Phamacol. Exp. Ther 166, 96 (1969)

Les rats mâles, Sprague Dawley de 200 à 300 g sont randomisés en lots de 10 et reçoivent les substances à tester par administration intra péritonéale 30 à 60 minutes avant l'injection locale au niveau de la patte arrière gauche de 0,1 ml d'une suspension à 0,5 % de Carragénine dans du sérum physiologique stérile.

La patte arrière droite servant de témoin, reçoit une injection de sérum physiologique.

Trois heures après l'injection de la Carragénine, les animaux sont anesthésiés de façon profonde au Narcorem **R** et le volume des deux pattes arrières mesuré par Pléthysmographie

Les résultats sont exprimés en pourcentage de variation de volume v% :

$$v\% = \frac{\text{volume patte gauche} - \text{volume patte droite}}{\text{volume patte droite}}$$

l'activité antiinflammatoire AAI% est définie par :

$$AAI\% = 100 - \left( \frac{v\%\ (\text{traité})}{v\%\ (\text{témoin})} \times 100 \right)$$

| Produits | Dose | AAI% |
|---|---|---|
| [3-méthoxycarbonyl 1,2,3,4-tétrahydro-β-carboline (3S)]-1-spiro cyclohexane | 50 mg/Kg | 14 % |
| [3-méthoxycarbonyl 1,2,3,4-tétrahydro-β-carboline (3S)]-1-spiro-4'-[1-méthyl pipéridine] | 25 mg/Kg | 14 % |

**EXEMPLE B : RECHERCHE DE L'ACTIVITE ANALGESIQUE**

Le potentiel analgésique de ces produits a été recherché selon le test dit "Acetic Acid Writhing" qui est basé sur le comptage des crampes abdominales induites chez la souris par injection intra péritonéale d'acide acétique (GAIRIN et Coll J. Pharmacol. Exp. Ther 245, 955 (1988))

Les souris randomisées en lots de 10 reçoivent les produits à tester par voie intra péritonéale 30 minutes avant l'administration intra péritonéale de 0,3 $cm^3$ d'acide acétique à 1%

Le nombre de crampes est compté entre la 5ème et 20ème minutes après l'injection d'acide acétique.

| Produits | Nombre de crampes par minute (entre la 5ème et la 20ème minutes) |
|---|---|
| - témoin | 23 ± 14 |
| - [3-méthoxycarbonyl 1,2,3,4-tétrahydro β carboline (3S)]-1-spiro cyclohexane 50 mg/Kg | 1 ± 1 |

Les composés de la littérature les plus proches structuralement des produits testés ne font pas preuve d'une telle activité analgésique.

**EXEMPLE C : RECHERCHE DE L'ACTIVITE ANXIOLYTIQUE**

Le potentiel anxiolytique de ces produits a été recherché selon le test "elevated plus maze".

Ce test permet d'étudier le comportement du rat, traité ou non, dans une situation de choix entre un espace clos rassurant ("bras clos") et un espace ouvert générateur d'anxiété ("bras ouvert").

Le test consiste à compter le nombre d'entrées dans chaque bras du labyrinthe durant 5 minutes et à mesurer le temps passé dans le "bras ouvert".

Les rats sont randomisés en lots de 8 et reçoivent les produits à tester par administration intrapéritonéale 60 minutes avant le début du test.

| Produits | Dose | nb total d'entrées | % d'entrées dans le bras ouvert | % du temps passé dans le bras ouvert |
|---|---|---|---|---|
| - témoin | | 14 ± 2 | 28 % ± 5 % | 22 % ± 5 % |
| - [3-méthoxycarbonyl 1,2,3,4-tétrahydro β carboline (3S)]-1-spiro cyclohexane | 50 mg/Kg IP | 18 ± 2 | 42 % ± 4 % | 40 % ± 6 % |
| - [3-méthoxycarbonyl 1,2,3,4-tétrahydro β carboline (3S)]-1-spiro-4'-[1-méthyl pipéridine] | 100mg/Kg IP | 18 ± 1 | 44 % ± 3 % | 41 % ± 4 % |

## Revendications

**1)** Indole substitué de formule générale **(I)** :

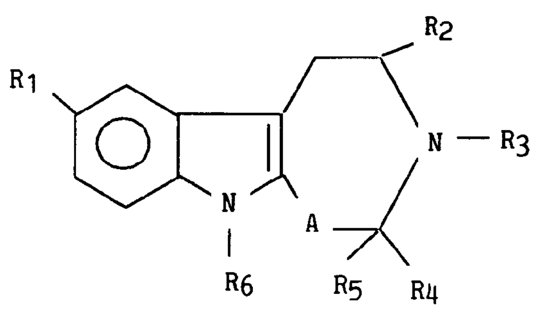

(I)

dans laquelle :

– A représente une liaison σ ou un groupement

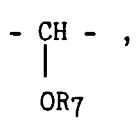

– $R_1$ représente un atome d'hydrogène ou d'halogène, un groupement hydroxy, alcoxy de 1 à 4 atomes de carbone linéaire ou ramifié, alkyle inférieur de 1 à 6 atomes de carbone linéaire ou ramifié éventuellement substitué par un ou des atomes d'halogènes ou groupements oxo, hydroxy, alcoxy de 1 à 4 atomes

de carbone,

– $R_2$ représente un groupement carboxy, un groupement alcoxy-carbonyle de 1 à 6 atomes de carbone ramifié ou non éventuellement substitué par un groupement phényle éventuellement substitué; un groupement phényloxycarbonyle éventuellement substitué, un groupement carbamoyle éventuellement substitué sur l'azote par un ou des groupements alkyle inférieur de 1 à 6 atomes de carbone linéaire ou ramifié ou cycloalkyle de 4 à 7 atomes de carbone et; dans le cas où A est un groupement de la forme

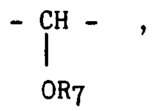

$R_2$ peut également être un atome d'hydrogène,

– $R_3$ représente un atome d'hydrogène, un groupement alkyle inférieur de 1 à 6 atomes de carbone ramifié ou non éventuellement substitué par un ou des atomes d'halogènes, ou groupements oxo, alcoxy inférieur de 1 à 4 atomes de carbone linéaire ou ramifié, phényle éventuellement substitué,

– $R_4$ et $R_5$ forment ensemble un système cyclique mono ou bicyclique saturé ou non, comprenant de 5 à 12 sommets pouvant comprendre dans le squelette cyclique de 0 à 3 hétéroatomes choisis parmi oxygène, azote, soufre et pouvant éventuellement être substitués par un ou des groupements oxo, alkyle inférieur de 1 à 6 atomes de carbone ramifié ou non, phényle éventuellement substitué, phénylcarbonyle éventuellement substitué, phénylalkyle de 7 à 9 atomes de carbone éventuellement substitué, fluorène éventuellement substitué avec, dans le cas où $R_4$ et $R_5$ forment un système bicyclique, la possibilité qu'un des cycles soit un noyau aromatique éventuellement substitué et, dans le cas où A est un groupement

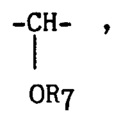

$R_4$ et $R_5$ peuvent également représenter chacun indépendamment l'un de l'autre un atome d'hydrogène, un groupement phényle éventuellement substitué, alkyle inférieur de 1 à 6 atomes de carbone ramifié ou non éventuellement substitué par 1 ou plusieurs groupements oxo, phényle éventuellement substitué, avec la réserve que, lorsque A est une liaison $\sigma$ avec $R_1 = R_3 = R_5 = H$ et $R_2 = COOH$ alors $R_4$ et $R_5$ ne peuvent former ensemble un cyclopentyle ou un cyclohexyle et, lorsque A est un groupement

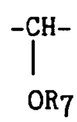

avec $R_7 = H$ ou

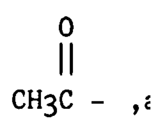

alors $R_4$ et $R_5$ doivent être différents de H,

– $R_6$ représente un atome d'hydrogène, un groupement alkyle inférieur de 1 à 6 atomes de carbone linéaire ou ramifié éventuellement substitué par un ou des groupements oxo, phényle éventuellement substitué,

– $R_7$ représente un atome d'hydrogène, un groupement alkyle de 1 à 6 atomes de carbone linéaire ou ramifié éventuellement substitué par un ou des groupements oxo, halogène, alcoxy de 1 à 4 atomes de carbone, cycloalkyle de 3 à 6 atomes de carbone, phényle éventuellement substitué,

– leurs isomères, diastéoisomères, énantiomères,

– leurs sels d'addition à un acide minéral ou organique pharmaceutiquement acceptable ou, dans le cas ou $R_2$ représente un groupement carboxy, leurs sels d'addition à une base minérale ou organique pharmaceutiquement acceptable,

– le terme substitué associé aux expressions phényle, phénylalkyle, phénylcarbonyle, phényloxycarbonyl signifie que les noyaux aromatiques peuvent être substitués par un ou des groupements alkyle de 1 à 6 atomes de carbone linéaire ou ramifié, alcoxy de 1 à 4 atomes de carbone, hydroxy, nitro, trifluorométhyle ou halogène,

**2)** Composés selon la revendication 1) pour lesquels A représente une liaison σ ce qui correspond aux 1,2,3,4-tétrahydro β carbolines de formule générale **(II)** :

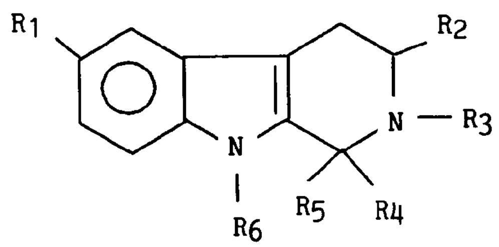

(II)

avec $R_1$, $R_2$, $R_3$, $R_4$, $R_5$ et $R_6$ ayant la même signification que dans la revendication 1),

– leurs isomères, diastéroisomères énantiomères,

– leurs sels d'addition à un acide minéral ou organique pharmaceutiquement acceptable, ou, dans le cas ou $R_2$ représente un groupement carboxylique, leurs sels d'addition à une base minérale ou organique pharmaceutiquement acceptable.

**3)** Composés selon la revendication 1) pour lesquels A représente un groupement

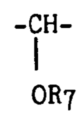

ce qui correspond aux 1,2,3,4,5,6- hexahydroazépino [4,5-b]indoles de formule générale **(III)** :

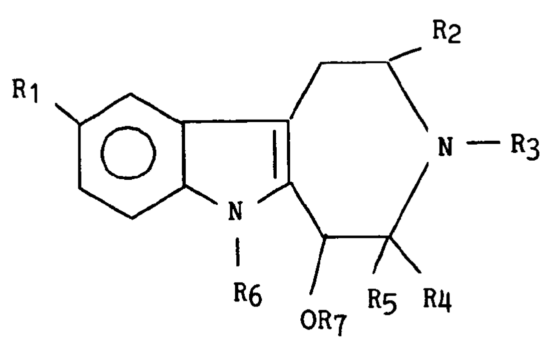

(III)

avec $R_1$, $R_2$, $R_3$, $R_4$, $R_5$, $R_6$ et $R_7$ ayant la même signification que dans la revendication 1)

– leurs isomères, diastéroisomères, énantiomères,

– leurs sels d'addition à un acide minéral ou organique pharmaceutiquement acceptable, ou dans le cas ou $R_2$ représente un groupement carboxylique, leurs sels d'addition avec une base minérale ou organique pharmaceutiquement acceptable.

**4)** Composés selon la revendication 1) avec $R_6$ étant un atome d'hydrogène et $R_3$ étant soit un atome d'hydrogène soit un groupement alkyle inférieur de 1 à 6 atomes de carbone ramifié ou non,

– leurs isomères, diastéréoisomères, énantiomères,

– leurs sels d'addition à un acide minéral ou organique pharmaceutiquement acceptable, ou, dans le cas

ou $R_2$ représente un groupement carboxylique, leurs sels d'addition à une base minérale ou organique pharmaceutiquement acceptable.

**5)** Composé selon la revendication 1) qui est le [3-méthoxycarbonyl 1,2,3,4-tétrahydro-β-carboline (3S)]-1-spiro-3'-[2-oxo 2,3-dihydroindole] ainsi que ses isomères et leurs sels d'addition à un acide pharmaceutiquement acceptable.

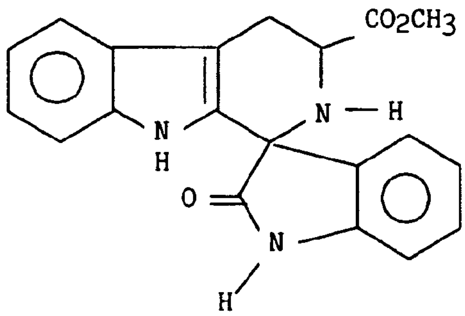

**6)** Composé selon la revendication 1) qui est le [3-méthoxycarbonyl 1,2,3,4-tétrahydro-β-carboline (3S)]-1-spiro cyclohexane ainsi que ses isomères et leurs sels d'addition à un acide pharmaceutiquement acceptable.

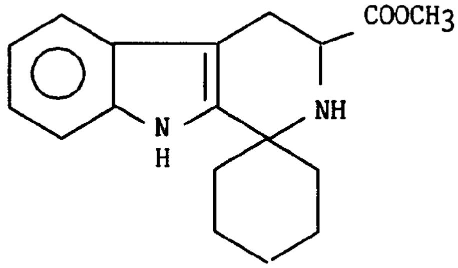

**7)** Composé selon la revendication 1) qui est le [3-méthoxycarbonyl 1,2,3,4-tétrahydro-β-carboline (3S)]-1-spiro cycloheptane ainsi que ses isomères et leurs sels d'addition à un acide pharmaceutiquement acceptable.

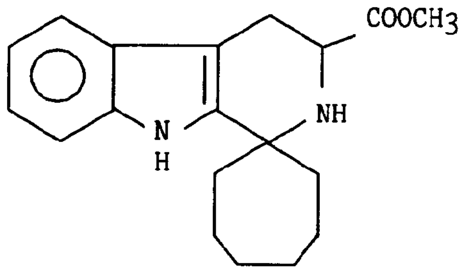

**8)** Composé selon la revendication 1) qui est le [3-méthoxycarbonyl 1,2,3,4-tétrahydro-β-carboline (3S)]-1-spiro-4'-pipéridine ainsi que ses isomères et leurs sels d'addition à un acide pharmaceutiquement acceptable.

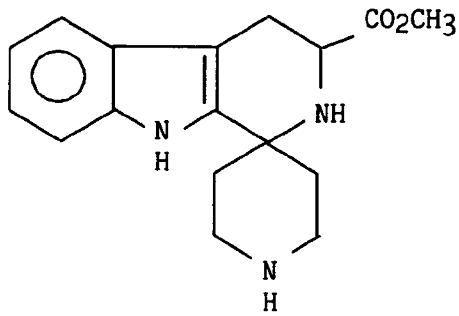

**9)** Composé selon la revendication 1) qui est le [3-méthoxycarbonyl 1,2,3,4-tétrahydro-β-carboline (3S)]-1-spiro-4'- [1'-méthyl pipéridine] ainsi que ses isomères et leurs sels d'addition à un acide pharmaceutiquement acceptable.

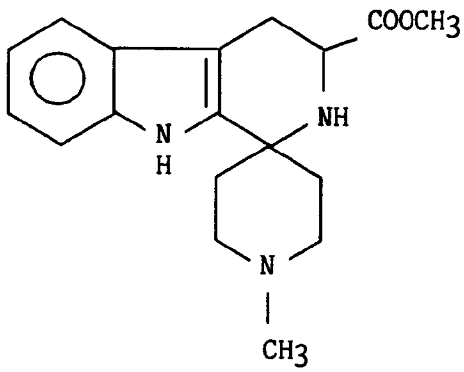

**10)** Composé selon la revendication 1) qui est le [3-carboxy 1,2,3,4-tétrahydro-β-carboline (3S)]-1-spiro-4'-cyclohexane ainsi que ses isomères et leurs sels d'addition à un acide ou une base pharmaceutiquement acceptable.

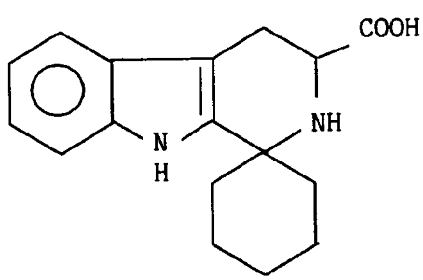

**11)** Composé selon la revendication 1) qui est le [3-carboxy 1,2,3,4-tétrahydro-β-carboline (3S)]-1-spiro-4'-pipéridine ainsi que ses isomères et leurs sels d'addition à un acide ou une base pharmaceutiquement acceptable.

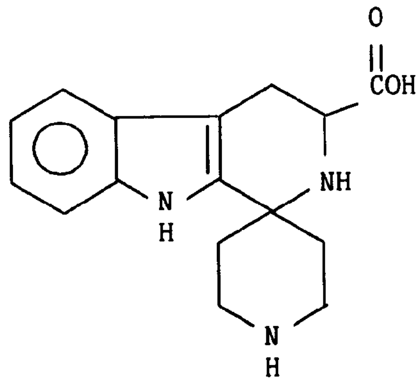

**12)** Composé selon la revendication 1) qui est le[3-carboxy 1,2,3,4-tétrahydro-β-carboline (3S)]-1-spiro-4′-[1′-méthyl pipéridine] ainsi que ses isomères et leurs sels d'addition à un acide ou à une base parmaceutiquement acceptables.

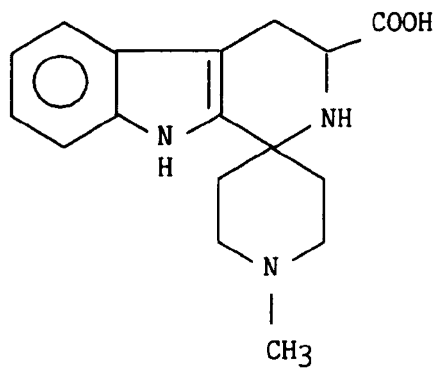

**13)** Composé selon la revendication 1) qui est le [3-méthoxycarbonyl 2-méthyl 1,2,3,4-tétrahydro-β-carboline (3S)]-1-spiro-4′-[1′-méthyl pipéridine] ainsi que ses isomères et leurs sels d'addition à un acide pharmaceutiquement acceptable.

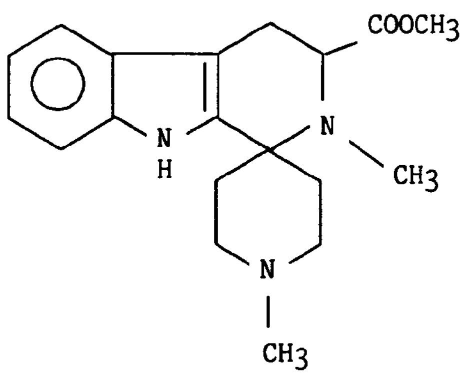

**14)** Composé selon la revendication 1) qui est le [3-carbamoyl 1,2,3,4-tétrahydro-β-carboline (3S)]-1-spiro-cyclohexane ainsi que ses isomères et leurs sels d'addition à un acide pharmaceutiquement acceptable.

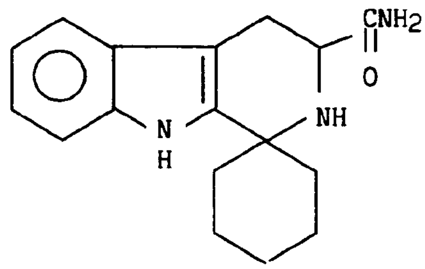

**15)** Composé selon la revendication 1) qui est le [3-(N-propyl carbamoyl) 1,2,3,4-tétrahydro-β-carboline (3S)]-1-spiro-cyclohexane ainsi que ses isomères et leurs sels d'addition à un acide pharmaceutiquement acceptable.

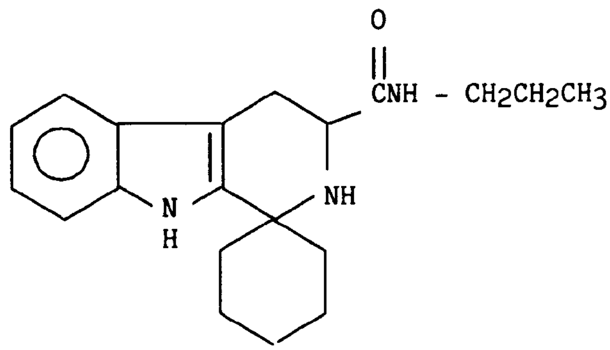

**16)** Composé selon la revendication 1) qui est le [3-(N-cyclohexyl carbamoyl) 1,2,3,4-tétrahydro-β-carboline (3S)]-1-spiro-cyclohexane ainsi que ses isomères et leurs sels d'addition à un acide pharmaceutiquement acceptable.

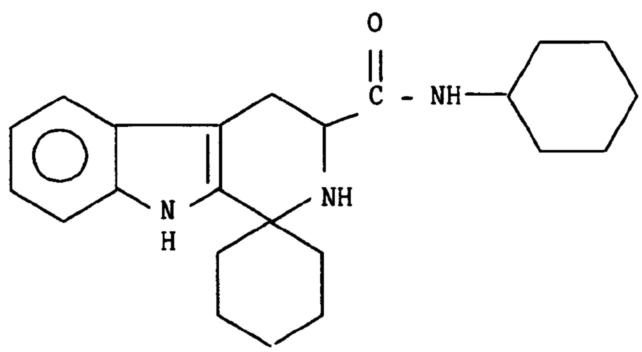

**17)** Composé selon la revendication 1) qui est le [5-acétoxy 1,2,3,4,5,6-hexahydroazépino [4,5-b] indole]-4-spiro-1'-(cyclohexène-3') ainsi que ses isomères et leurs sels d'addition à un acide pharmaceutiquement acceptable.

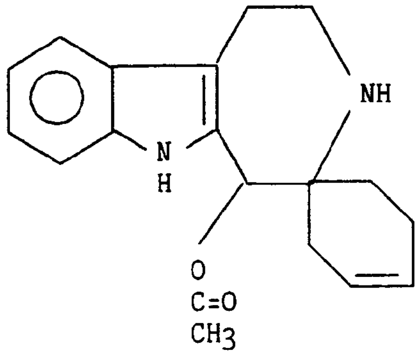

**18)** Composé selon la revendication 1) qui est le [5-méthoxy 1,2,3,4,5,6-hexahydroazépino [4,5-b] indole]-4-spiro-1'-(cyclohexène-3') ainsi que ses isomères et leurs sels d'addition à un acide pharmaceutiquement acceptable.

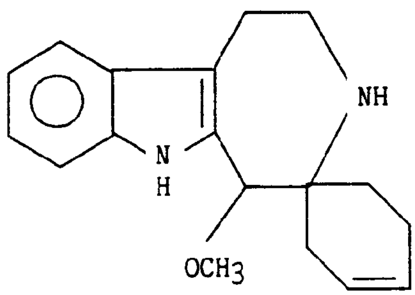

**19)** Composé selon la revendication 1) qui est le [ 5-acétoxy 9-méthoxy 1,2,3,4,5,6-hexahydroazépino [4,5-b] indole]-4-spiro-1'-(cyclohexène-3') ainsi que ses isomères et leurs sels d'addition à un acide pharmaceutiquement acceptable.

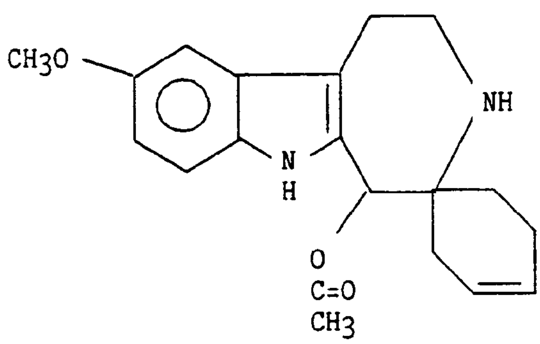

**20)** Composé selon la revendication 1) qui est le [5-acétoxy 3-méthyl 1,2,3,4,5,6-hexahydroazépino [4,5-b] indole]-4-spiro-1'-(cyclohexène-3') ainsi que ses isomères et leurs sels d'addition à un acide pharmaceutiquement acceptable.

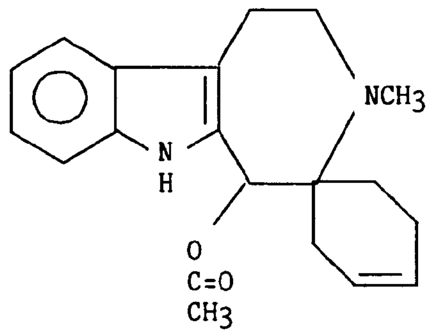

**21)** Composé selon la revendication 1) qui est le 5-acétoxy 9-méthoxy 4,4-diméthyl 1,2,3,4,5,6-hexahydroazépino [4,5-b] indole ainsi que ses isomères et leurs sels d'addition à un acide pharmaceutiquement acceptable.

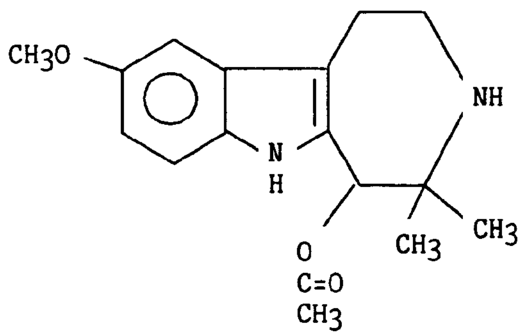

**22)** Composé selon la revendication 1) qui est le 5-hydroxy 4,4-diméthyl 1,2,3,4,5,6-hexahydroazépino [4,5-b] indole ainsi que ses isomères et leurs sels d'addition à un acide pharmaceutiquement acceptable.

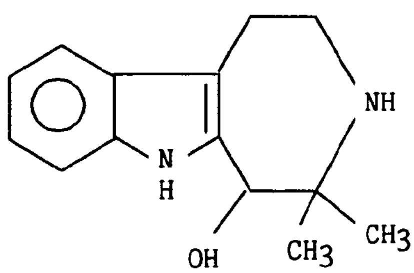

**23)** Procédé de préparation des composés de la revendication 1) qui varie selon la nature du groupement A,

– soit l'on fait réagir une tryptamine substituée de formule générale **(IV)** :

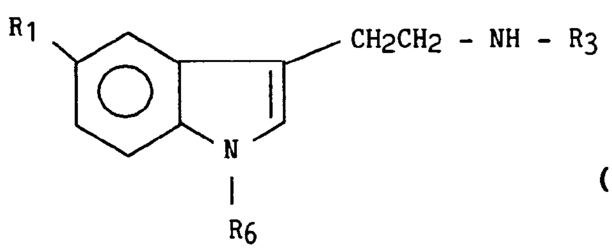

**(IV)**

dans laquelle $R_1$, $R_3$ et $R_6$ ont la même signification que dans le composé de formule générale **(I)** avec un aldéhyde de formule générale **(V)** :

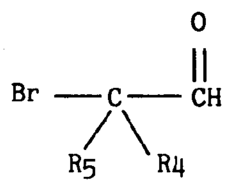

(V)

dans laquelle $R_5$ et $R_4$ ont la même signification que dans le composé de formule générale **(I)** en les chauffant dans un acide organique de formule générale **(VI)** :

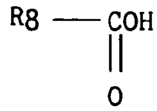

(VI)

dans laquelle $R_8$ est un groupement méthyl, éthyl, trifluorométhyl de manière à obtenir le 1,2,3,4,5,6-hexahydroazépino [4,5-b] indole de formule générale **(VII)** :

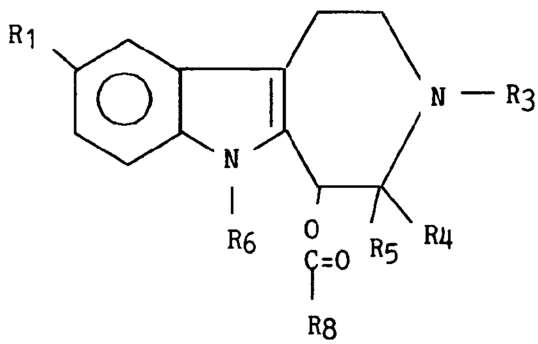

(VII)

dans laquelle $R_1$, $R_3$, $R_4$, $R_5$, $R_5$ ont la même signification que dans le composé de formule générale **(I)** et $R_5$ la même signification que dans l'acide **(VI)** que l'on peut, soit chauffer en présence d'un alcool de formule générale $R_9OH$ dans laquelle $R_9$ est un groupement alkyle inférieur de 1 à 6 atomes de carbones ramifié ou non ou un groupement aralkyle de manière à obtenir le 1,2,3,4,5,6-hexahydroazépino [4,5-b] indole de formule générale **(VIII)** :

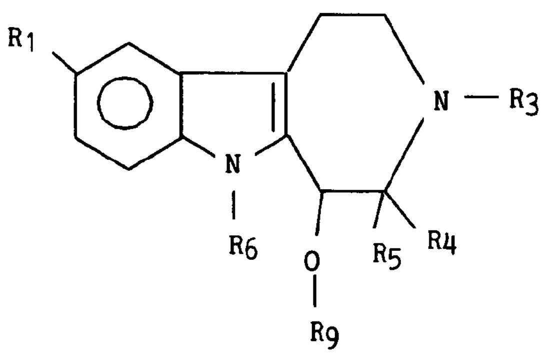

(VIII)

dans laquelle $R_1$, $R_3$, $R_4$, $R_5$, $R_5$ ont la même signification que dans le composé de formule générale **(I)** et $R_9$ la même signification que ci-dessus, soit chauffer dans du méthanol en présence de 10 équivalents de carbonate de potassium de manière à obtenir le 1,2,3,4,5,6-hexahydroazépino [4,5-b] indole de formule générale **(IX)** :

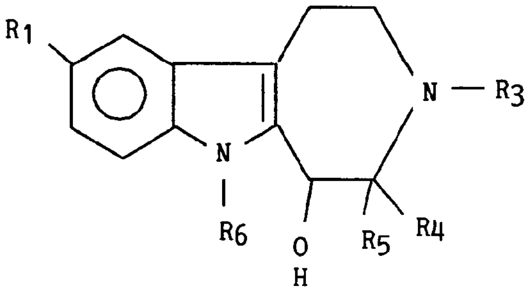

**(IX)**

dans laquelle $R_1$, $R_3$, $R_4$, $R_5$, $R_6$ ont la même signification que dans le composé de formule générale **(I)**,

– soit l'on fait réagir un tryptophane substitué de formule générale **(X)** :

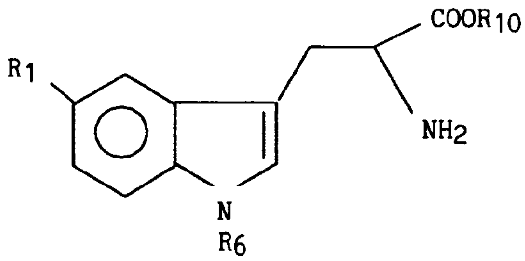

**(X)**

dans laquelle $R_1$, $R_6$ ont la même définition que dans le composé de formule générale **(I)** et $R_{10}$ est un alkyle inférieur de 1 à 6 atomes de carbones ramifié ou non ou un groupement aryle ou aralkyle éventuellement substitué avec une cétone de formule générale **(XI)** :

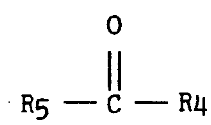

**(XI)**

dans laquelle $R_4$ et $R_6$ ont la même signification que dans le composé de formule générale **(I)** sous atmosphère d'azote soit dans du toluène ou du benzène au reflux en présence d'acide tosylique, l'eau formée lors de la condensation étant éliminée au moyen d'un appareil extracteur d'eau pouvant être par exemple un Dean Stark, soit dans certains cas, au reflux du méthanol ou de la cétone elle même, de manière à obtenir après purification la β-carboline de formule générale **(XII)**

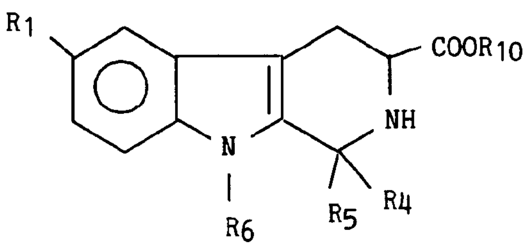

**(XII)**

dans laquelle $R_1$, $R_4$, $R_5$, $R_6$, $R_{10}$ ont la même signification que dans les composés de formule générale **(I)** et **(X)** que l'on peut :

– soit faire réagir dans des conditions d'amination réductrice avec un aldéhyde de formule générale **(XIII)** :

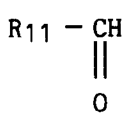

**(XIII)**

dans laquelle $R_{11}$ est un atome d'hydrogène ou un groupement alkyle inférieur de 1 à 6 atomes de carbones ramifié ou non éventuellement substitué par un ou des atomes d'halogènes ou des groupements alcoxy inférieur de 1 à 4 atomes de carbone ou aryle substitués ou non de manière à obtenir la β carboline de formule générale **(XIV)** :

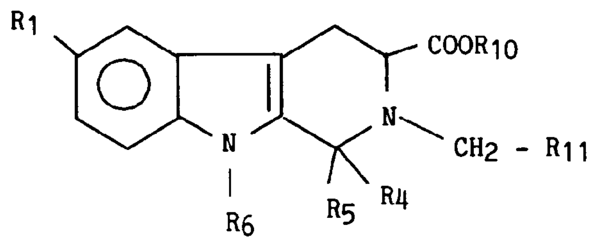

**(XIV)**

dans laquelle $R_1$, $R_{10}$, $R_{11}$, $R_4$, $R_5$, $R_6$ ont la même signification que dans les composés de formule générale **(XII)** et **(XIII).**

– soit faire réagir avec un chlorure d'acide, ou l'anhydride correspondant, de formule générale **(XV):**

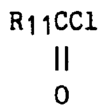

**(XV)**

dans laquelle $R_{11}$ a la même signification que dans le composé de formule générale **(XIII)** de manière à obtenir la β-carboline de formule générale **(XVI)** :

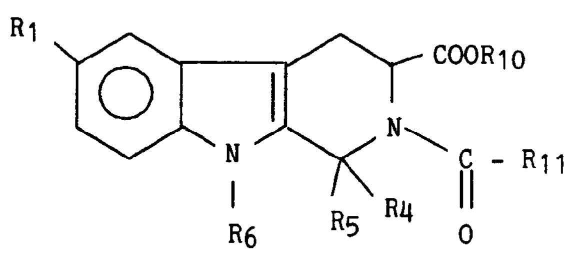

**(XVI)**

dans laquelle $R_1$, $R_{10}$, $R_{11}$, $R_6$, $R_5$, $R_4$ ont la même signification que dans le composé de formule générale **(XIV),**

– soit faire réagir, dans le cas où $R_{10} = CH_3$, avec une solution aqueuse d'hydroxyde de Baryum de manière à obtenir les acides de formule générale **(XVII)** :

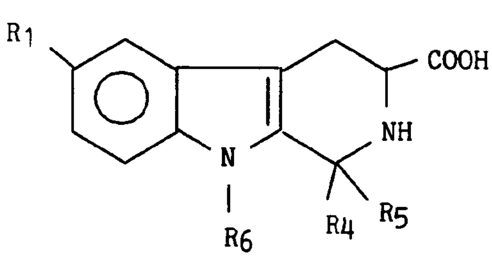

**(XVII)**

dans laquelle $R_1$, $R_4$, $R_5$ et $R_6$ ont la même signification que dans les composés de formule générale **(I),**

– soit faire réagir dans le cas où $R_{10} = CH_3$, avec une amine de formule générale **(XVIII)** :

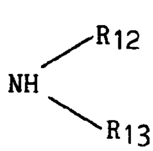

**(XVIII)**

dans laquelle $R_{12}$ et $R_{13}$ représentent chacun indépendamment l'un de l'autre un atome d'hydrogène ou des groupements alkyles inférieurs de 1 à 6 atomes de carbone ramifié ou non ou cycloalkyle de 4 à 7 atomes de carbone, de manière à obtenir les amides de formule générale **(XIX)** :

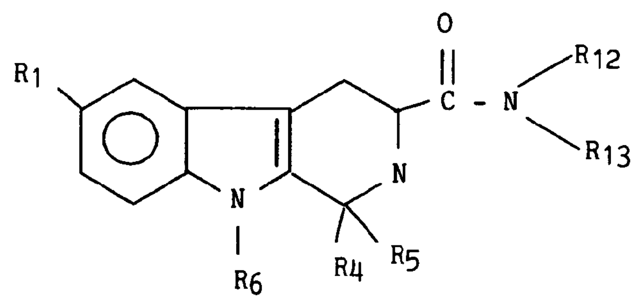

**(XIX)**

dans laquelle $R_1$, $R_4$, $R_5$, $R_5$ ont la même signification que dans les composés de formule générale **(I)** et $R_{12}$ et $R_{13}$ la même signification que dans les amides de formule générale **(XVIII),**
étant entendu que les composés de formule générale VII, VIII, IX, XII, XIV, XVI, XVII,XIX font partie de l'invention et sont des cas particuliers des composés de formule générale **(I)**

**24)** Compositions pharmaceutiques contenant comme principe actif un composé selon la revendication 1) seul ou en combinaison avec un ou plusieurs excipients ou véhicules inertes non toxiques pharmaceutiquement acceptables.

**25)** Compositions pharmaceutiques selon la revendication 24) présentées sous une forme convenant notamment pour le traitement des troubles du système nerveux central, de la mémoire, de la douleur, de l'inflammation , des accidents vasculaires cérébraux et des convulsions.

**Revendications pour les Etats contractants suivants : GR ES**

**1)** Procédé de préparation d'indole substitué de formule générale **(I)** :

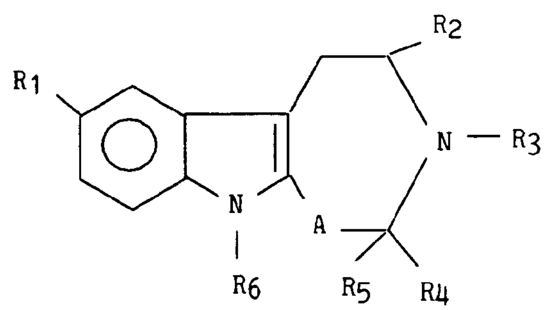

**(I)**

dans laquelle :
– A représente une liaison σ ou un groupement

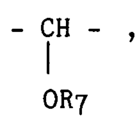

– $R_1$ représente un atome d'hydrogène ou d'halogène, un groupement hydroxy, alcoxy de 1 à 4 atomes de carbone linéaire ou ramifié, alkyle inférieur de 1 à 6 atomes de carbone linéaire ou ramifié éventuellement substitué par un ou des atomes d'halogènes ou groupements oxo, hydroxy, alcoxy de 1 à 4 atomes de carbone,
– $R_2$ représente un groupement carboxy, un groupement alcoxycarbonyle de 1 à 6 atomes de carbone ramifié ou non éventuellement substitué par un groupement phényle éventuellement substitué, un groupement phényloxycarbonyle éventuellement substitué, un groupement carbamoyle éventuellement substitué sur l'azote par un ou des groupements alkyle inférieur de 1 à 6 atomes de carbone linéaire ou ramifié ou

cycloalkyle de 4 à 7 atomes de carbone et, dans le cas ou A est un groupement de la forme

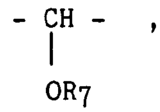

$R_2$ peut également être un atome d'hydrogène,

– $R_3$ représente un atome d'hydrogène, un groupement alkyle inférieur de 1 à 6 atomes de carbone ramifié ou non éventuellement substitué par un ou des atomes d'halogènes, ou groupements oxo, alcoxy inférieur de 1 à 4 atomes de carbone linéaire ou ramifié, phényle éventuellement substitué,

– $R_4$ et $R_5$ forment ensemble un système cyclique mono ou bicyclique saturé ou non, comprenant de 5 à 12 sommets pouvant comprendre dans le squelette cyclique de 0 à 3 hétéroatomes choisis parmi oxygène, azote, soufre et pouvant éventuellement être substitués par un ou des groupements oxo, alkyle inférieur de 1 à 6 atomes de carbone ramifié ou non, phényle éventuellement substitué, phénylcarbonyle éventuellement substitué, phénylalkyle de 7 à 9 atomes de carbone éventuellement substitué, fluorène éventuellement substitué avec, dans le cas ou $R_4$ et $R_5$ forment un système bicyclique, la possibilité qu'un des cycles soit un noyau aromatique éventuellement substitué et, dans le cas ou A est un groupement

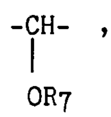

$R_4$ et $R_5$ peuvent également représenter chacun indépendamment l'un de l'autre un atome d'hydrogène, un groupement phényle éventuellement substitué, alkyle inférieur de 1 à 6 atomes de carbone ramifié ou non éventuellement substitué par 1 ou plusieurs groupements oxo, phényle éventuellement substitué, avec la réserve que, lorsque A est une liaison $\sigma$ avec $R_1 = R_3 = R_5 = H$ et $R_2 = COOH$ alors $R_4$ et $R_5$ ne peuvent former ensemble un cyclopentyle ou un cyclohexyle et, lorsque A est un groupement

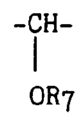

avec $R_7 = H$ ou

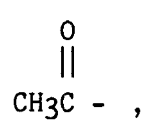

alors $R_4$ et $R_5$ doivent être différents de H,

– $R_6$ représente un atome d'hydrogène, un groupement alkyle inférieur de 1 à 6 atomes de carbone linéaire ou ramifié éventuellement substitué par un ou des groupements oxo, phényle éventuellement substitué,

– $R_7$ représente un atome d'hydrogène, un groupement alkyle de 1 à 6 atomes de carbone linéaire ou ramifié éventuellement substitué par un ou des groupements oxo, halogène, alcoxy de 1 à 4 atomes de carbone, cycloalkyle de 3 à 6 atomes de carbone, phényle éventuellement substitué,

(le terme substitué associé aux expressions phényle, phénylalkyle, phénylcarbonyle signifie que les noyaux aromatiques peuvent être substitués par un ou des groupements alkyle de 1 à 6 atomes de carbone linéaire ou ramifié, alcoxy de 1 à 4 atomes de carbone, hydroxy, nitro, trifluorométhyle ou halogène,)

ainsi que de leurs isomères, diastéoisomères, énantiomères et de leurs sels d'addition à un acide minéral ou organique pharmaceutiquement acceptable ou, dans le cas ou $R_2$ représente un groupement carboxy, leurs sels d'addition à une base minérale ou organique pharmaceutiquement acceptable,
Caractérisé en ce que :
– Soit l'on fait réagir une tryptamine substituée de formule générale **(IV)** :

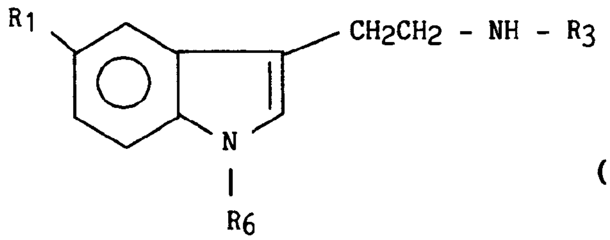

(IV)

dans laquelle $R_1$, $R_3$ et $R_6$ ont la même signification que dans le composé de formule générale **(I)** avec un aldéhyde de formule générale **(V)** :

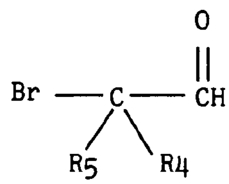

(V)

dans laquelle $R_5$ et $R_4$ ont la même signification que dans le composé de formule générale **(I)** en les chauffant dans un acide organique de formule générale **(VI)** :

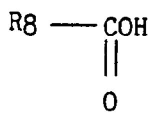

(VI)

dans laquelle $R_8$ est un groupement méthyl, éthyl, trifluorométhyl de manière à obtenir le 1,2,3,4,5,6-hexahydroazépino [4,5-b] indole de formule générale **(VII)** :

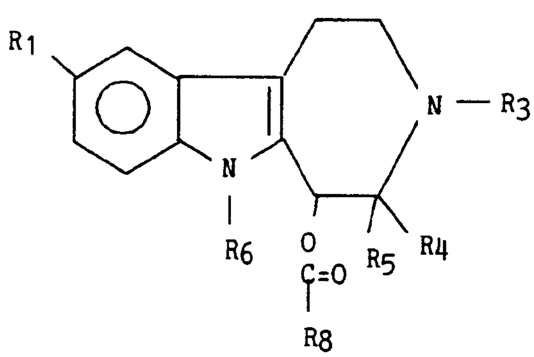

(VII)

dans laquelle $R_1$, $R_3$, $R_4$, $R_6$, $R_6$ ont la même signification que dans le composé de formule générale **(I)** et $R_6$ la même signification que dans l'acide **(VI)** que l'on peut, soit chauffer en présence d'un alcool de formule générale $R_9OH$ dans laquelle $R_9$ est un groupement alkyle inférieur de 1 à 6 atomes de carbones ramifié ou non ou un groupement aralkyle de manière à obtenir le 1,2,3,4,5,6-hexahydroazépino [4,5-b] indole de formule générale **(VIII)** :

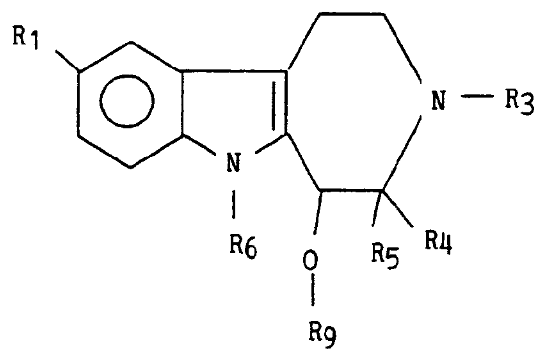

(VIII)

dans laquelle $R_1$, $R_3$, $R_4$, $R_5$, $R_5$ ont la même signification que dans le composé de formule générale **(I)** et $R_9$ la même signification que ci-dessus, soit chauffer dans du méthanol en présence de 10 équivalents de carbonate de potassium de manière à obtenir le 1,2,3,4,5,6-hexahydroazépino [4,5-b] indole de formule générale **(IX)** :

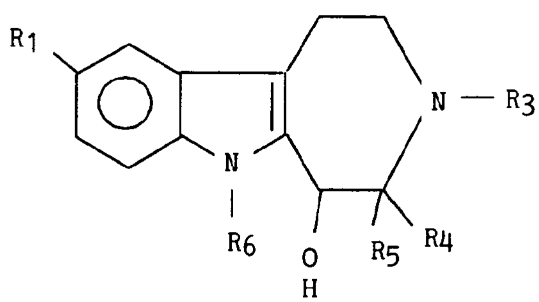

(IX)

dans laquelle $R_1$, $R_3$, $R_4$, $R_5$, $R_5$ ont la même signification que dans le composé de formule générale **(I)**,

– soit l'on fait réagir un tryptophane substitué de formule générale **(X)** :

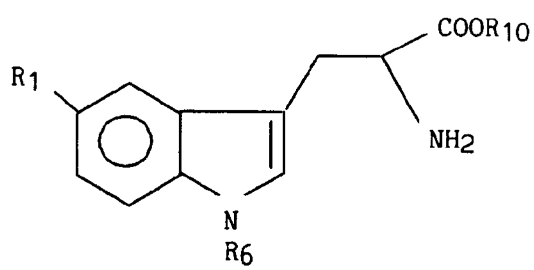

(X)

dans laquelle $R_1$, $R_6$ ont la même définition que dans le composé de formule générale **(I)** et $R_{10}$ est un alkyle inférieur de 1 à 6 atomes de carbone ramifié ou non ou un groupement aryle ou aralkyle éventuellement substitué avec une cétone de formule générale **(XI)** :

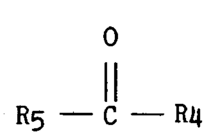

(XI)

dans laquelle $R_4$ et $R_5$ ont la même signification que dans le composé de formule générale **(I)** sous atmosphère d'azote soit dans du toluène ou du benzène au reflux en présence d'acide tosylique, l'eau formée lors de la condensation étant éliminée au moyen d'un appareil extracteur d'eau pouvant être par exemple un Dean Stark, soit, dans certains cas, au reflux du méthanol ou de la cétone elle même, de manière à obtenir après purification la β carboline de formule générale **(XII)** :

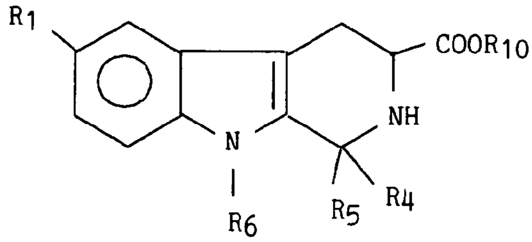

**(XII)**

dans laquelle $R_1$, $R_4$, $R_5$, $R_6$, $R_{10}$ ont la même signification que dans les composés de formule générale **(I)** et **(X)** que l'on peut :

– soit faire réagir dans des conditions d'amination réductrice avec un aldéhyde de formule générale **(XIII)** :

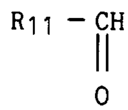

**(XIII)**

dans laquelle $R_{11}$ est un atome d'hydrogène ou un groupement alkyle inférieur de 1 à 6 atomes de carbone ramifié ou non éventuellement substitué par un ou des atomes d'halogènes ou des groupements alcoxy ou aryle substitués ou non de manière à obtenir la β carboline de formule générale **(XIV)** :

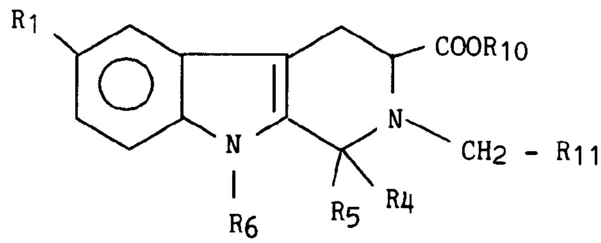

**(XIV)**

dans laquelle $R_1$, $R_{10}$, $R_{11}$, $R_4$, $R_6$, $R_6$ ont la même signification que dans les composés de formule générale **(XII)** et **(XIII),**

– soit faire réagir avec un chlorure d'acide, ou l'anhydride correspondant, de formule générale **(XV):**

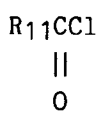

**(XV)**

dans laquelle $R_{11}$ a la même signification que dans le composé de formule générale **(XIII)** de manière à obtenir la β carboline de formule générale **(XVI)** :

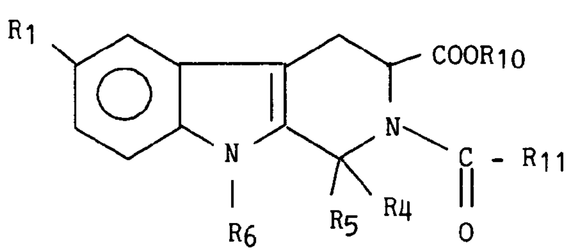

**(XVI)**

dans laquelle $R_1$, $R_{10}$, $R_{11}$, $R_6$, $R_6$, $R_4$ ont la même signification que dans le composé de formule générale **(XIV),**

– soit faire réagir, dans le cas ou $R_{10} = CH_3$, avec une solution aqueuse d'hydroxyde de Baryum de manière à obtenir les acides de formule générale **(XVII)** :

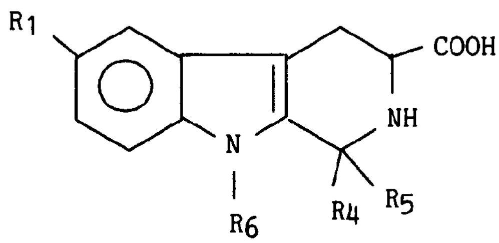

(XVII)

dans laquelle $R_1$, $R_4$, $R_5$ et $R_6$ ont la même signification que dans les composés de formule générale **(I)**,

– soit faire réagir dans le cas ou $R_{10} = CH_3$, avec une amine de formule générale **(XVIII)** :

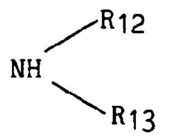

(XVIII)

dans laquelle $R_{12}$ et $R_{13}$ représentent chacun indépendamment l'un de l'autre un atome d'hydrogène ou des groupements alkyles inférieurs de 1 à 6 atomes de carbone ramifié ou non ou cycloalkyle de 4 à 7 atomes de carbone, de manière à obtenir les amides de formule générale **(XIX)** :

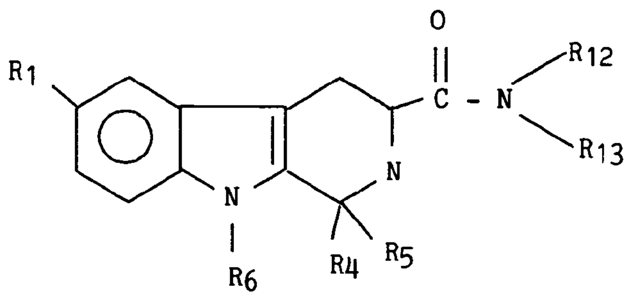

(XIX)

dans laquelle $R_1$, $R_4$, $R_5$, $R_5$ ont la même signification que dans les composés de formule générale **(I)** et $R_{12}$ et $R_{13}$ la même signification que dans les amides de formule générale **(XVIII)**,

**2)** Procédé de préparation selon la revendication 1) des composés de formule générale **(II)** :

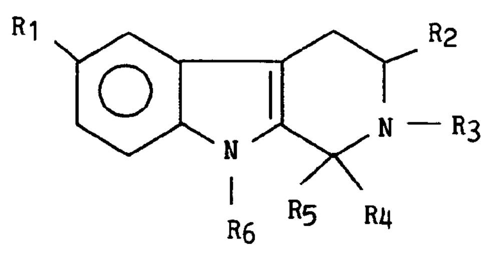

(II)

avec $R_1$, $R_2$, $R_3$, $R_4$, $R_5$ et $R_5$ ayant la même signification que dans la revendication 1), ainsi que de leurs isomères, diastéroisomères énantiomères et de leurs sels d'addition à un acide minéral ou organique pharmaceutiquement acceptable, ou, dans le cas ou $R_2$ représente un groupement carboxylique, de leurs sels d'addition à une base minérale ou organique pharmaceutiquement acceptable.,

**3)** Procédé de préparation selon la revendication 1) des composés de formule générale **(III)** :

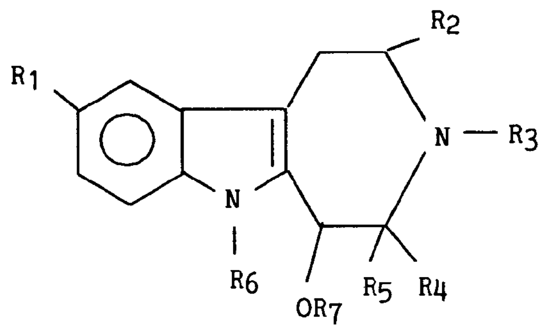

(III)

avec $R_1$, $R_2$, $R_3$, $R_4$, $R_5$, $R_5$ et $R_7$ ayant la même signification que dans la revendication 1), ainsi que de leurs isomères, diastéroisomères, énantiomères et de leurs sels d'addition à un acide minéral ou organique pharmaceutiquement acceptable, ou dans le cas ou $R_2$ représente un groupement carboxylique, de leurs sels d'addition avec une base minérale ou organique pharmaceutiquement acceptable.

**4)** Procédé de préparation selon les revendications 1) et 2) du composé qui est le [3-méthoxycarbonyl 1,2,3,4-tétrahydro-β-carboline (3S)]-1-spiro-3'-[2-oxo 2,3-dihydroindole] ainsi que de ses isomères et leurs sels d'addition à un acide pharmaceutiquement acceptable.

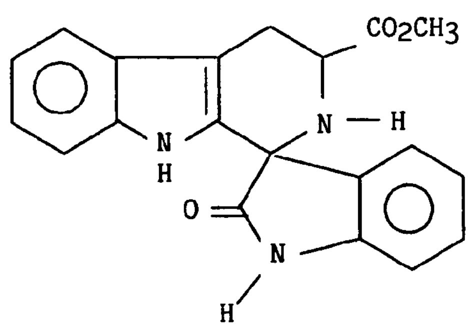

**5)** Procédé de préparation selon les revendications 1) et 2) du composé qui est le [3-méthoxycarbonyl 1,2,3,4-tétrahydro-β-carboline (3S)]-1-spiro cyclohexane ainsi que de ses isomères et de leurs sels d'addition à un acide pharmaceutiquement acceptable.

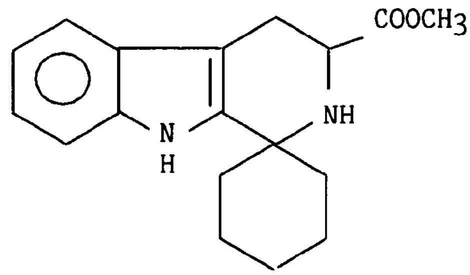

6) Procédé de préparation selon les revendications 1) et 2) du composé qui est le [3-méthoxycarbonyl 1,2,3,4-tétrahydro-β-carboline (3S)]-1-spiro cycloheptane ainsi que de ses isomères et de leurs sels d'addition à un acide pharmaceutiquement acceptable.

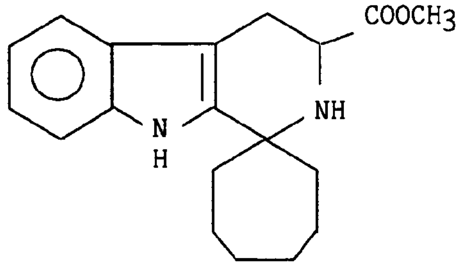

7) Procédé de préparation selon les revendications 1) et 2) du composé qui est le [3-méthoxycarbonyl 1,2,3,4-tétrahydro-β-carboline (3S)]-1-spiro-4'-pipéridine ainsi que de ses isomères et de leurs sels d'addition à un acide pharmaceutiquement acceptable.

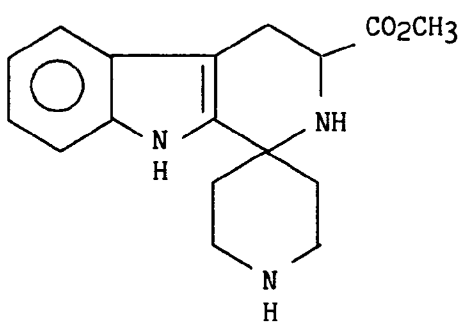

8) Procédé de préparation selon les revendications 1) et 2) du composé qui est le [3-méthoxycarbonyl 1,2,3,4-tétrahydro-β-carboline (3S)]-1-spiro-4'- [1'-méthyl pipéridine] ainsi que de ses isomères et de leurs sels d'addition à un acide pharmaceutiquement acceptable.

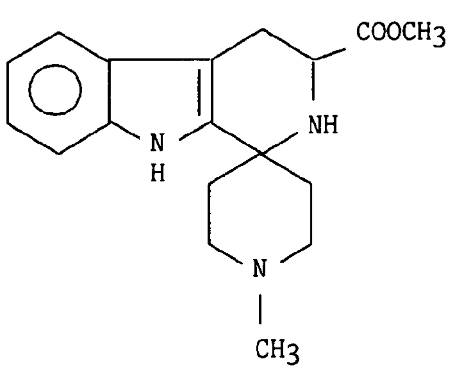

**9)** Procédé de préparation selon les revendications 1) et 2) du composé qui est le [3-carboxy 1,2,3,4-tétrahydro-β-carboline (3S)]-1-spiro-4′-cyclohexane ainsi que de ses isomères et de leurs sels d'addition à un acide ou une base pharmaceutiquement acceptable.

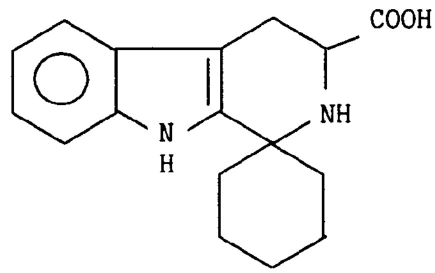

**10)** Procédé de préparation selon les revendications 1) et 2) du composé qui est le [3-carboxy 1,2,3,4-tétrahydro-β-carboline (3S)]-1-spiro-4′-pipéridine ainsi que de ses isomères et de leurs sels d'addition à un acide ou une base pharmaceutiquement acceptable.

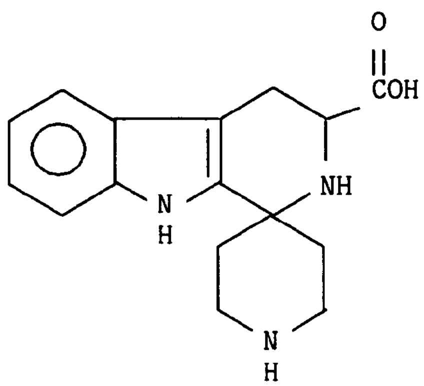

**11)** Procédé de préparation selon les revendications 1) et 2) du composé qui est le[3-carboxy 1,2,3,4-tétrahydro-β-carboline (3S)]-1-spiro-4′-[1′-méthyl pipéridine] ainsi que de ses isomères et de leurs sels d'addition à un acide ou à une base parmaceutiquement acceptables.

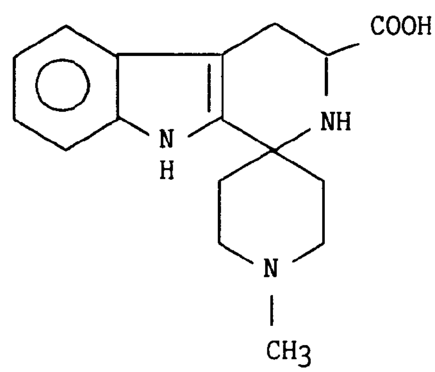

**12)** Procédé de préparation selon les revendications 1) et 2) du composé qui est le [3-méthoxycarbonyl 2-méthyl 1,2,3,4-tétrahydro-β-carboline (3S)]-1-spiro-4′-[1′-méthyl pipéridine] ainsi que de ses isomères et de leurs sels d'addition à un acide pharmaceutiquement acceptable.

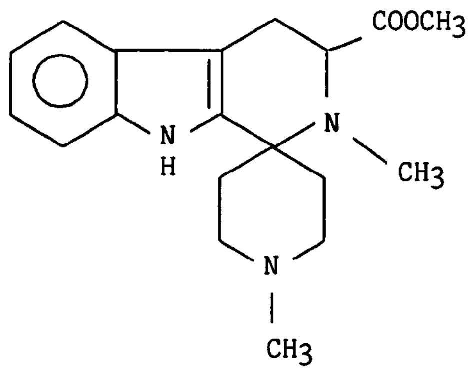

**13)** Procédé de préparation selon les revendications 1) et 2) du composé qui est le [3-carbamoyl 1,2,3,4-tétrahydro-β-carboline (3S)]-1-spiro-cyclohexane ainsi que de ses isomères et de leurs sels d'addition à un acide pharmaceutiquement acceptable.

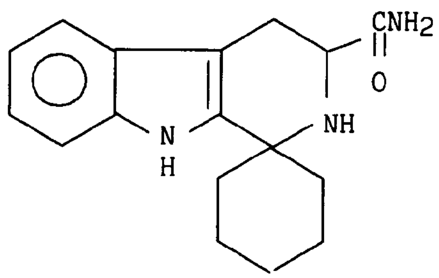

**14)** Procédé de préparation selon les revendications 1) et 2) du composé qui est le [3-(N-propyl carbamoyl) 1,2,3,4-tétrahydro-β-carboline (3S)]-1-spiro-cyclohexane ainsi que de ses isomères et de leurs sels d'addition à un acide pharmaceutiquement acceptable.

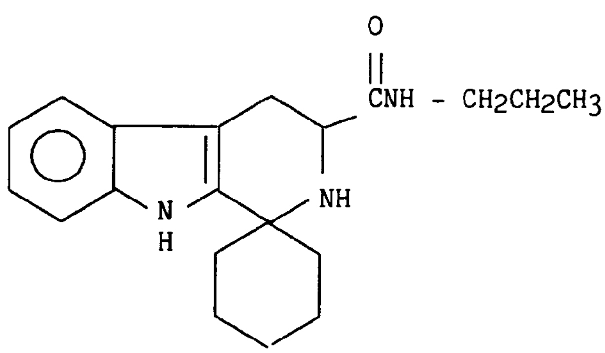

**15)** Procédé de préparation selon les revendications 1) et 2) du composé qui est le [3-(N-cyclohexyl carbamoyl) 1,2,3,4-tétrahydro-β-carboline (3S)]-1-spiro-cyclohexane ainsi que de ses isomères et de leurs sels d'addition à un acide pharmaceutiquement acceptable.

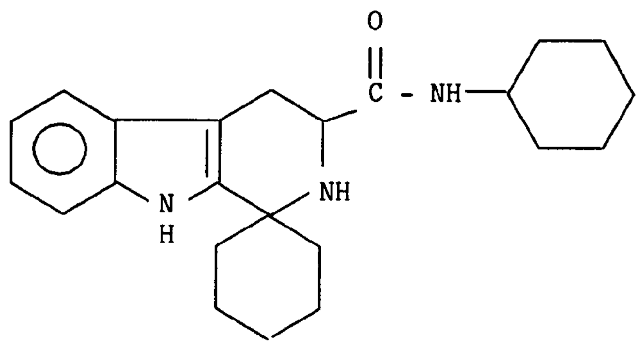

**16)** Procédé de préparation selon les revendications 1) et 3) du composé qui est le [5-acétoxy 1,2,3,4,5,6-hexahydroazépino [4,5-b] indole]-4-spiro-1′-(cyclohexène-3′) ainsi que de ses isomères et de leurs sels d'addition à un acide pharmaceutiquement acceptable.

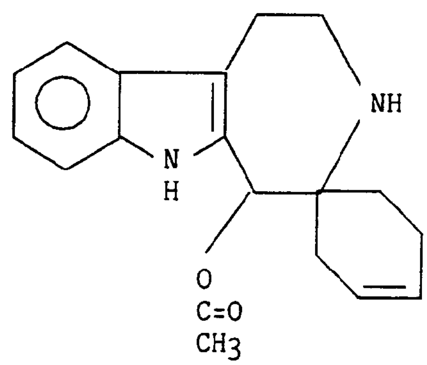

**17)** Procédé de préparation selon les revendications 1) et 3) du composé qui est le [5-méthoxy 1,2,3,4,5,6-hexahydroazépino [4,5-b] indole]-4-spiro-1′-(cyclohexène-3′) ainsi que de ses isomères et de leurs sels d'addition à un acide pharmaceutiquement acceptable.

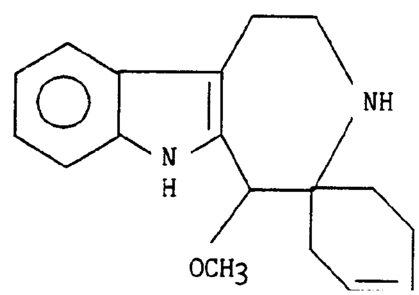

**18)** Procédé de préparation selon les revendications 1) et 3) du composé qui est le [ 5-acétoxy 9-méthoxy 1,2,3,4,5,6-hexahydroazépino [4,5-b] indole]-4-spiro-1′-(cyclohexène-3′) ainsi que de ses isomères et de leurs sels d'addition à un acide pharmaceutiquement acceptable.

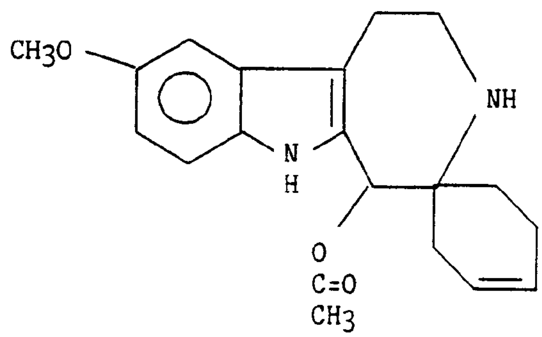

**19)** Procédé de préparation selon les revendications 1) et 3) du composé qui est le [5-acétoxy 3-méthyl 1,2,3,4,5,6-hexahydroazépino [4,5-b] indole]-4-spiro-1′-(cyclohexène-3′) ainsi que de ses isomères et de leurs sels d'addition à un acide pharmaceutiquement acceptable.

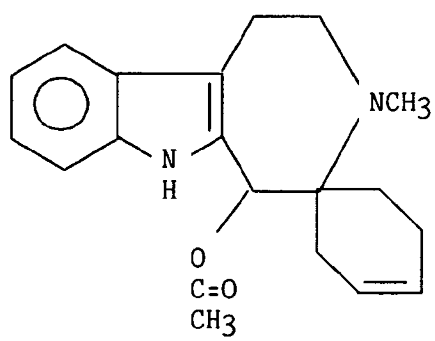

**20)** Procédé de préparation selon les revendications 1) et 3) du composé qui est le 5-acétoxy 9-méthoxy 4,4-diméthyl 1,2,3,4,5,6-hexahydroazépino [4,5-b] indole ainsi que de ses isomères et de leurs sels d'addition à un acide pharmaceutiquement acceptable.

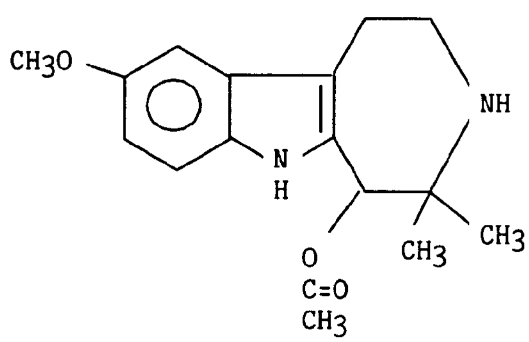

**21)** Procédé de préparation selon les revendications 1) et 3) du composé qui est le 5-hydroxy 4,4-diméthyl 1,2,3,4,5,6-hexahydroazépino [4,5-b] indole ainsi que de ses isomères et de leurs sels d'addition à un acide pharmaceutiquement acceptable.

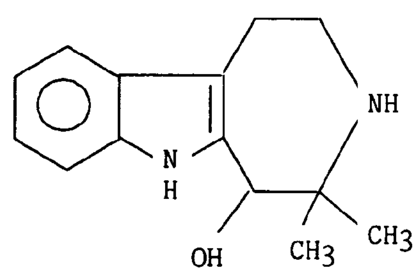

**22)** Procédé de préparation des compositions pharmaceutiques contenant comme principe actif un dérivé préparé selon les revendications 1) à 21) seul ou en combinaison avec un ou plusieurs excipients ou véhicules inertes non toxiques pharmaceutiquement acceptables.

**23)** Préparation de compositions pharmaceutiques selon la revendication 22) présentées sous une forme convenant notamment pour le traitement des troubles du système nerveux central, de la mémoire, de la douleur, de l'inflammation , des accidents vasculaires cérébraux et des convulsions.

Office européen des brevets

# RAPPORT DE RECHERCHE EUROPEENNE

Numero de la demande

EP 91 40 1740
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A,D | MONATSHEFTE FÜR CHEMIE, vol. 116, no. 6/7, 1985, pages 851 - 855; E.M. AFSAH et al.: "Pictet-Spengler Reactions of Tryptamine and Tryptophan with Cycloalkanones and Ketonic Mannich Bases" * page 852, schéma des réactions * * pages 854, 855, partie expérimentale * --- | 1, 2, 4, 6, 23 | C07D471/04 C07D487/04 C07D471/20 A61K31/435 A61K31/55 //(C07D471/04, 221:00,209:00) (C07D487/04, 223:00,209:00) (C07D471/20, 221:00,221:00, 209:00) |
| A | EP-A-0021857 (SYNTHELABO) * page 3 * * page 14, ligne 30 - page 16, ligne 5 * --- | 1, 2, 23-25 | |
| A,D | EP-A-0028381 (SANDOZ AG) * revendications 1, 2, 6-8 * --- | 1, 3, 24, 25 | |
| A,D | EP-A-0203902 (OMNICHEM S.A.) * revendications 1, 17, 19 * --- | 1, 3, 24, 25 | |
| A,D | FR-A-1524830 (J.R. GEIGY S.A.) * page 1, ligne 1 - page 2, ligne 11 * * page 3, alinéa 3 - page 4, alinéa 2 * --- | 1, 3, 24 | DOMAINES TECHNIQUES RECHERCHES (Int. Cl.5 ) |
| A | DE-A-1695943 (UPJOHN CO.) * page 1, ligne 1 - page 6, ligne 18 * | 1, 3, 24 | C07D471/00 C07D487/00 |
| D | & FR-A-1524495 --- | | |
| A | DE-B-1244793 (UPJOHN CO.) * colonne 1, lignes 1 - 47 * --- | 1, 2, 23 | |
| A | BULLETIN DE LA SOCIETE CHIMIQUE DE FRANCE, no. 6, 1973, pages 2058 - 2064; M. JULIA et al.: "Tétrahydro- et dihydro-ß-carbolines dérivées du tryptophane et du méthoxy-5 tryptophane" * page 2059, tableau II; page 2063, lignes 5 - 8 * --- | 1, 2, 23 | |
| | -/-- | | |

Le présent rapport a été établi pour toutes les revendications

| Lieu de la recherche | Date d'achèvement de la recherche | Examinateur |
|---|---|---|
| BERLIN | 11 SEPTEMBRE 1991 | HASS C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 91 40 1740
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | PATENT ABSTRACTS OF JAPAN<br>vol. 8, no. 205 (C-243)(1642) 19 septembre 1984,<br>& JP-A-59 95286 (OOTSUKA SEIYAKU KOUJIYOU K.K.)<br>01 juin 1984,<br>* le document en entier *<br>----- | 16-18 | |

DOMAINES TECHNIQUES RECHERCHES (Int. Cl.5 )

Le présent rapport a été établi pour toutes les revendications

| Lieu de la recherche | Date d'achèvement de la recherche | Examinateur |
|---|---|---|
| BERLIN | 11 SEPTEMBRE 1991 | HASS C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)